# EUROPEAN PATENT APPLICATION

(11) **EP 4 079 852 A1**
(43) Date of publication of application: **26.10.2022**
(21) Application number: 20903934.6
(22) Date of filing: 18.12.2020
(51) Int. Cl.: C12N 15/09, A01K 67/027

(54) **NON-HUMAN PRIMATE ALZHEIMER'S DISEASE MODEL ANIMAL AND METHOD FOR PRODUCING SAME**

(30) Priority: 20.12.2019 US 201962951498 P
(71) Applicant: RIKEN, Wako-shi, Saitama 351-0198 (JP); Central Institute For Experimental Animals, Kawasaki-shi, Kanagawa 210-0821 (JP)
(72) Inventor: SASAKI, Erika, kawasaki-shi, Kanagawa 210-0821 (JP); SATO, Kenya, kawasaki-shi, Kanagawa 210-0821 (JP); KUMITA, Wakako, kawasaki-shi, Kanagawa 210-0821 (JP); SASAGURI, Hiroki, Wako-shi, Saitama 351-0198 (JP); SAIDO, Takaomi, Wako-shi, Saitama 351-0198 (JP); NAGATA, Kenichi, Wako-shi, Saitama 351-0198 (JP); YAMAMOTO, Takashi, Hiroshima-shi, Hiroshima 739-8511 (JP); SAKUMA, Tetsushi, Hiroshima-shi, Hiroshima 739-8511 (JP)
(74) Representative: Graf von Stosch Patentanwaltsgesellschaft mbH
(86) International application number: PCT/JP2020/047548
(87) International publication number: WO 2021/125349

(57) **Abstract**

Provided is an animal model that can replicate a disease state of human AD. A non-human primate model animal of Alzheimer's disease includes the *PSEN1* gene in which a site related to splicing of exon 9 is made deficient.

## Description

### Technical Field

The present invention relates to a non-human primate model animal of Alzheimer's disease and a method for producing the model animal.

### Background Art

Alzheimer's disease (AD) is a major cause of dementia, with the number of patients with AD anticipated to exceed 50 million worldwide. However, no effective measures have been established to prevent or treat AD, which is due in part to a lack of useful animal models. Some mice models have been established, but cannot reflect human AD disease states in most parts due to biological differences from primates.

### Citation List

### Non-Patent Literatures

Non-Patent Literature 1
   Sasaguri H. et al. APP mouse models for Alzheimer's disease preclinical studies. EMBO J 1;36(17):2473-2487, doi: 10.15252/embj.201797397 (2017).
Non-Patent Literature 2
   Saito T. et al. Single App knock-in mouse models of Alzheimer's disease. Nat Neurosci. 17(5):661-663. doi:10.1038/nn.3697 (2014).
Non-Patent Literature 3
   Oakley H et al. Intraneuronal beta-amyloid aggregates, neurodegeneration, and neuron loss in transgenic mice with five familial Alzheimer's disease mutations: potential factors in amyloid plaque formation. J Neurosci. 26(40):10129-10140. doi:10.1523/JNEUROSCI.1202-06. (2006).

### Summary of Invention

### Technical Problem

An objective of the present invention is to provide a world's first non-human primate AD model.

### Solution to Problem

To attain the above objective, the inventors performed various experiments and found that mutant *PSEN1* marmoset individuals whose 3'-splice site (acceptor site) of exon 9 in the *PSEN1* gene (that encodes presenilin 1 protein) was partly or completely deleted by Transcription Activator-Like Effector Nuclease (TALEN) in marmoset embryos and individuals could replicate a pathological status observed in human AD patients. On the basis of this finding, the inventors completed the present invention. Specifically, the present invention includes the following features.
(1) A non-human primate model animal of Alzheimer's disease, including: the *PSEN1* gene in which a site including at least a 5'- or 3'-splice site of exon 9 and being related to splicing of exon 9 is deficient.
(2) The non-human primate model animal of Alzheimer's disease described in (1), wherein the non-human primate is a marmoset.
(3) A method for producing a non-human primate model animal of Alzheimer's disease, the method including: making a site of the *PSEN1* gene deficient by a genome-editing technology, the site including at least a 5'- or 3'-splice site of exon 9 in the *PSEN1* gene and being related to splicing of exon 9.
(4) The method described in (3), wherein the site including at least the 5'- or 3'-splice site of exon 9 in the *PSEN1* gene and being related to splicing of exon 9 is made deficient in an ovum.
(5) The method described in (3) or (4), wherein the non-human primate is a marmoset.

In another aspect, the present invention includes the following features:
(6) A non-human primate model animal of Alzheimer's disease including: the *PSEN1* gene in which an acceptor site of exon 9 is deficient.
(7) The non-human primate model animal of Alzheimer's disease described in (6), wherein the non-human primate is a marmoset.
(8) A method for producing a non-human primate model animal of Alzheimer's disease, the method including: making an acceptor site of exon 9 in the *PSEN1* gene deficient by a genome-editing technology.
(9) The method described in (8), wherein the acceptor site of exon 9 in the *PSEN1* gene is made deficient in an ovum.

### Advantageous Effects of Invention

In accordance with an aspect of the present invention, it is possible to replicate a human AD disease state.

### Brief Description of Drawings

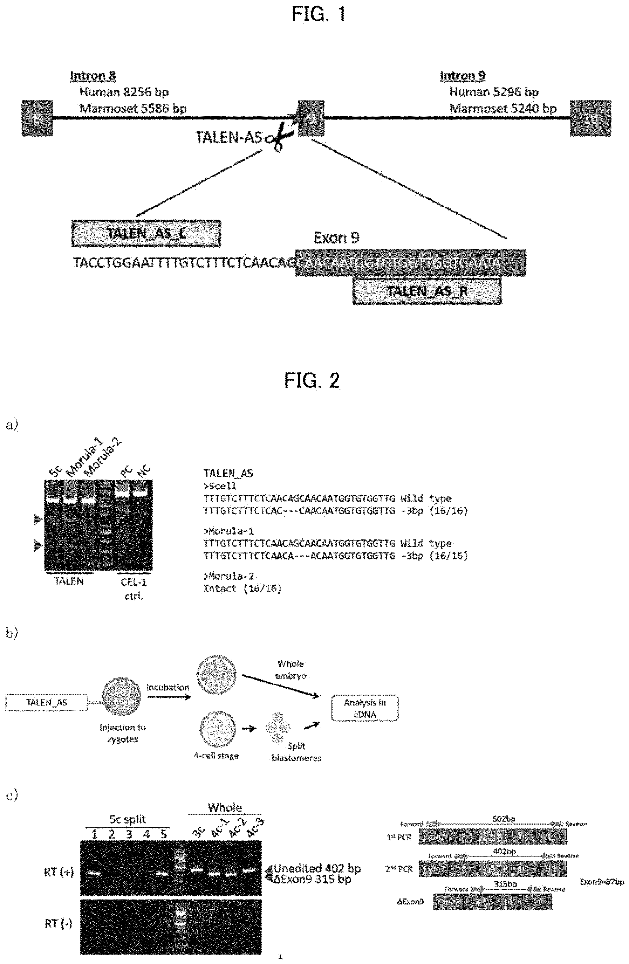

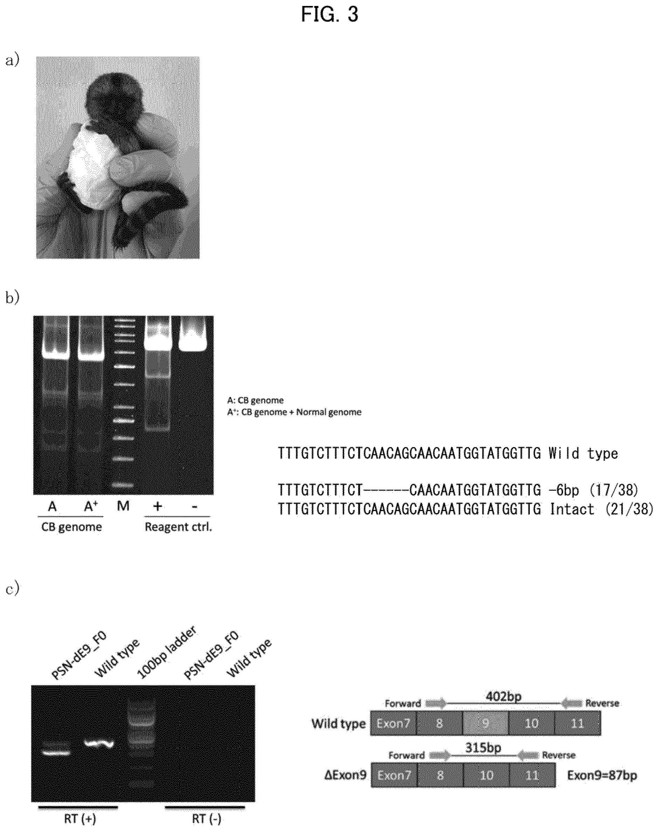

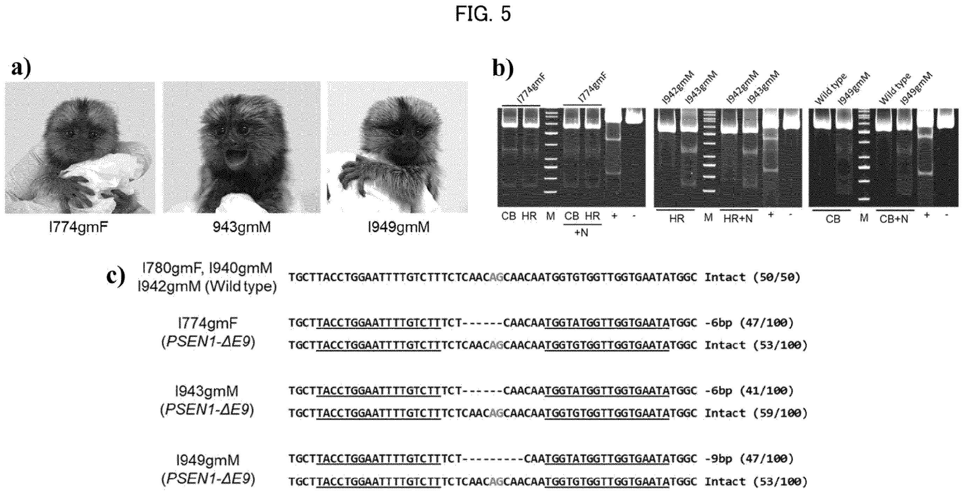

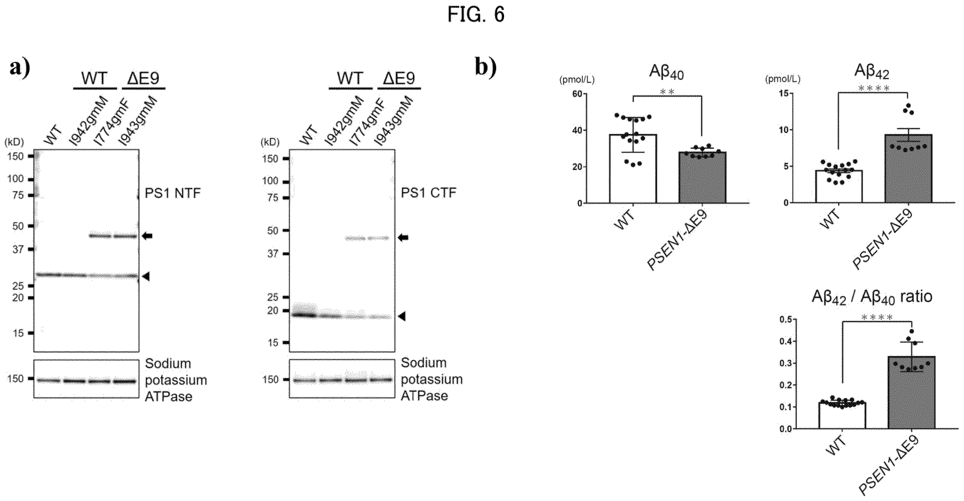

Fig. 1 is a conceptual view schematically illustrating an example of Platinum TALEN, which is utilizable in the present invention, targeting the 3'-splice site of exon 9 in a marmoset. In Fig. 1, the regions surrounded by the quadrangular frames correspond to TALEN target sites and exons in the marmoset *PSEN1* gene, and the numbers surrounded by the quadrangular frames indicate exon numbers. The 3'-splice site ("AG") of exon 9 is located upstream of exon 9.
a of Fig. 2 shows a result of surveyor assay carried out, in an Example, after injection of TALEN in the marmoset fertilized ova (three left columns; "PC" on the right side is a positive control, and "NC" on the right side is a negative control). b of Fig. 2 is a view schematically illustrating an outline of TALEN injection and subsequent *PSEN1* mRNA analysis, which were carried out in the Example. c of Fig. 2 shows a result of reverse transcription PCR (RT-PCR) on the fertilized ova after TALEN injection, which was carried out in the Example.
a of Fig. 3 is a photograph of a neonate of a marmoset AD model in accordance with an aspect of the present invention, which was obtained in an Example. b of Fig. 3 shows a result of surveyor assay carried out using genomic DNAs extracted from cord blood of the neonate. c of Fig. 3 shows a result of PCR carried out using RNAs extracted from hair roots of the marmoset neonate and a wild-type marmoset.

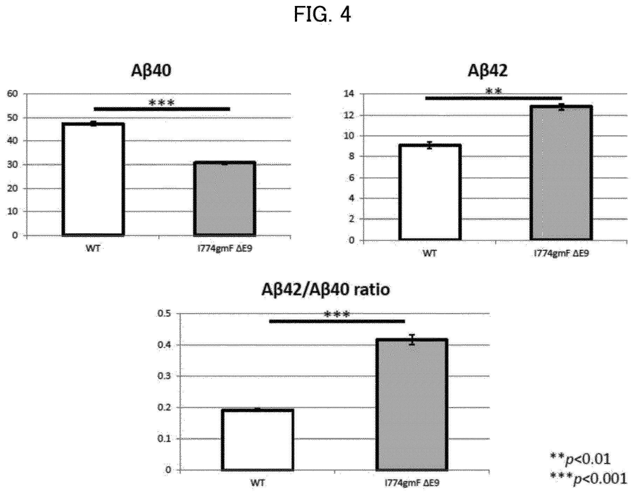

Fig. 4 shows a result of quantification of two kinds of amyloid β proteins in the fibroblasts collected from ear tissues of mutant marmosets (8 month old) obtained in an Example.
a of Fig. 5 is a photograph of a neonate of a marmoset AD model in accordance with an aspect of the present invention, obtained in Example 4. b of Fig. 5 shows a result of surveyor assay carried out using genomic DNAs extracted from cord blood of the neonate. c of Fig. 5 shows sequences of wild-type and mutant neonate genomes around the 3'-splice site of exon 9.
a of Fig. 6 shows a result of Western blot analysis carried out using homogenates of fibroblasts and antibodies to PS1 proteins (left: N-terminal fragment (PS1 NTF), right: C-terminal fragment (PS1 CTF)). b of Fig. 6 shows a result of quantification of two kinds of amyloid β proteins in the fibroblasts collected from the ear tissues of the mutant marmosets (8 month old) obtained in the Example. All the fibroblasts were collected from the ear tissues of the mutant marmosets (8 month old) obtained in Example 4.

### Description of Embodiments

The present invention relates to non-human primate models of AD. There is no particular limitation on the kind of non-human primate, provided that the primate can have Alzheimer's disease. Examples of the primate encompass *Strepsirrhini* (e.g., lemurs, lorises, and galagos) and *Haplorhini* (e.g., tarsiers, spider monkeys, capuchin monkeys, marmosets, guenons, colobi, and anthropoid apes). However, this is not limitative. In a preferred aspect, examples of the non-human primates encompass guenons (e.g., rhesus monkeys and crab-eating macaques) and marmosets (e.g., common marmosets and black-eared marmosets). A marmoset AD model in accordance with an aspect of the present invention is advantageous for AD research for the following reasons: marmosets have a genetic background and a brain structure close to those of humans; marmosets exhibit human-like cognitive behaviors associated with prefrontal cortex; marmosets are able to communicate by visual and auditory information: marmosets have a small body size (e.g., 350 to 500 g); marmosets have a relatively short gestation period (145 days) and their reproductive efficiency is high (40 to 80 neonates per female); and marmosets have a relatively long life span (10 to 13 years in captivity). Marmosets are advantageous also for the following reasons: marmosets exhibit deposition of amyloid β protein with aging starting around 7 years; and the marmoset amyloid β protein has a sequence identical to that of human amyloid β protein.

A marmoset AD model in accordance with an aspect of the present invention has the *PSEN1* gene in which a splice site of exon 9 is destroyed. The destruction of the splice site may be carried out by a known genome-editing technology, examples of which encompass CRISPR-Cas9 system and Transcription Activator-Like Effector Nuclease (TALEN). In an aspect, the destruction is preferably carried out by Platinum TALEN. It has been reported that a deletion mutation in exon 9 or a point mutation at a splice site of exon 9 in the *PSEN1* gene instigates exclusion of exon 9 in an mRNA due to alternative splicing. The inventors are the first to actually succeed in production of a non-human primate AD model by destruction of this site.

Fig. 1 is a conceptual view of Platinum TALEN targeting the 3'-splice site of exon 9 in the marmoset *PSEN1* gene. Each of TALEN_AS_L indicated on the left and TALEN_AS_R indicated on the right in Fig. 1 can be constructed by a known method including use of Platinum Gate TALEN kit. Each of TALEN_AS_L and TALEN_AS_R is a TALEN target site. A region of a sequence surrounded by a quadrangular frame corresponds to exon of the marmoset *PSEN1* gene. Bold-faced "AG" adjacent to exon 9 on the upstream side corresponds to the 3'-splice site of exon 9. The 5'-splice site is "GT" (not illustrated) adjacent to exon 9 on the downstream side.

In a preferred embodiment, the expression "3'-splice site of exon 9 in the *PSEN1* gene" indicates two nucleotides existing at the 3'-terminal of intron 8 for splicing of exon 8 and exon 9, as shown in Fig. 1. The expression "deficiency of the 3'-splice site of exon 9 in the *PSEN1* gene" means functional deficiency of the 3'-splice site. The deficiency is caused by deletion of one or both of the two nucleotides (only nucleotide A, only nucleotide G, or both nucleotides AG) (see Figs. 2 and 3). In the embodiment, the "deficiency of a site including at least the 3'-splice site of exon 9 in the *PSEN1* gene (i.e., the site being related to splicing of exon 9)" is essentially caused by the deletion.

The deficiency is any of (i) partial deletion of the 3'-terminal (including the above-described nucleotides AG) of intron 8 and the 5'-terminal of exon 9, (ii) partial deletion of the 3'-terminal (including the above-described nucleotides AG or nucleotide A) of intron 8, and (iii) partial deletion of the nucleotide G or nucleotides AG and the 5'-terminal of exon 9. The full length of the deletion causing the deficiency may be arbitrarily selected, provided that the deletion includes one or both of the two nucleotides and does not give any effect on splicing of any other exon than exon 9.

In another embodiment different from the preferred embodiment shown in Fig. 1, the deficiency may be caused by deletion of the 5'-splice site of exon 9 in the *PSEN1* gene. The meaning of the deficiency in this embodiment can be understood by the preceding two paragraphs in which the following parts are replaced as below.
1. The "3'-splice site" may be replaced with "5'-splice site".
2. The "exon 8 and exon 9" may be replaced with "exon 9 and exon 10".
3. The "3'-terminal of intron 8" may be replaced with "5'-terminal of intron 9".
4. The "5'-terminal of exon 9" may be replaced with "3'-terminal of exon 9".
5. The "nucleotide A" may be replaced with "nucleotide G", the "nucleotide G" may be replaced with "nucleotide T", and "nucleotides AG" may be replaced with "nucleotides GT".

In another embodiment different from the above two embodiments, the "deficiency of a site including at least the 5'-splice site or the 3'-splice site of exon 9 in the *PSEN1* gene (the site being related to splicing of exon 9)" can be caused by deletion of a branch site existing in intron. The intron can be either of or both intron 8 and intron 9 in the *PSEN1* gene. It is known that the branch site exits near the 3'-terminal in the intron and is followed by, on its downstream side, a polypyrimidine region (repetition of Py). In an aspect of the present invention, the branch site can be a site including nucleotide A located 20 to 40 (said to be 21 to 34 nucleotides) nucleotides upstream of the 3'-splice site. The deletion of the branch site means deletion of at least the above-described nucleotide A.

Note that a genome of a marmoset AD model in accordance with an aspect of the present invention may have all of or any two of the above-described three different deletions.

A method for producing a marmoset AD model in accordance with an aspect of the present invention is, for example, as follows. TALEN mRNAs targeting the 3'-splice site of exon 9 in the marmoset *PSEN1* gene are introduced into the nuclei of marmoset fertilized ova. The resulting products may be subcloned, followed by surveyor assay and sequencing analysis. Alternatively, only embryos in which a target site including the 3'-splice site is deficient may be collected. The deficiency can be induced at a high possibility, e.g., two out of three. After the introduction of TALEN, RT-PCR and cDNA sequencing can be carried out to confirm exclusion of exon 9 in the *PSEN1* mRNA. According to this method, there is no wild-type sequence in an RNA extracted from a 4-cell-stage fertilized ovum or a single blastomere, and the 3'-splice site can be made deficient in a biallelic manner.

Preferably, TALEN mRNAs are introduced into ova, and then the ova are fertilized. After fertilization, the fertilized ova are developed to a 6-cell stage or more. Then, the fertilized ova can be transferred to surrogate mothers. The introduction of TALEN does not cause any distinct adverse reaction in pregnancy or following development. In this manner, it is possible to obtain marmosets carrying, e.g., a 6-bp deficiency including the 3'-splice site in a heterozygous state. It is possible to confirm exclusion of exon 9 by carrying out, on mRNAs from the obtained marmoset AD models (e.g., the mRNAs extracted from hair roots of the marmosets), RT-PCR and then cDNA sequencing.

As indicated in the later-described Examples, a marmoset AD model in accordance with an aspect of the present invention exhibits an increase of Aβ₄₂/Aβ₄₀ ratio compared to that of a wild-type marmoset, where Aβ₄₂ is amyloid β protein Aβ₄₂ and Aβ₄₀ is amyloid β protein Aβ₄₀. For example, a marmoset AD model in accordance with an aspect of the present invention has a value of Aβ₄₂/Aβ₄₀ that is 1.4-fold or more, 1.6-fold or more, 1.8-fold or more, or 2-fold or more greater than that of the wild-type.

### Examples

Examples are indicated below. However, the present invention is not limited to these Examples.

All the animal experiments described below were conducted in accordance with the guidelines for animal experimentation as determined by the Central Institute for Experimental Animals on the basis of "the Act on Welfare and Management of Animals", "Standard relating to the Care and Keeping and Reducing Pain of Laboratory Animals" and "Fundamental Guidelines for Proper Conduct of Animal Experiment and Related Activities in Academic Research Institutions (notified by the Ministry of Education, Culture, Sports, Science and technology of Japan)".

### Materials and method

### Preparation of TALEN plasmids

Platinum Gate TALEN Kit (Addgene) was used to prepare TALEN plasmids in accordance with a method described in SCIENTIFIC REPORTS 2013, Nov 29; 3: 3379. Doi:10.1038/srep03379. This method will be briefly explained below. Platinum Gate TALEN kit (Addgene; cat#1000000043) was used to carry out two-step Golden Gate assembly method, so as to construct Platinum TALEN containing a homodimer-type FokI nuclease domain. The assembled repeat arrays were subsequently inserted into a final destination vector, ptCMV-153/47-VR. TALEN mRNA was synthesized with use of "mMESSAGE mMACHINE T7 Ultra Transcription Kit" (Thermo Fisher Scientific, AM1345). Transcribed mRNA was purified with use of "MEGAclear Transcription Clean-Up Kit" (Thermo Fisher Scientific, AM1908). The full-length sequences of the plasmids thus prepared are indicated as SEQ ID NO: 1 (Left_TALEN) and SEQ ID NO: 2 (Right_TALEN) in the sequence listing.

### Sequences of TALEN vectors

SEQ ID NO: 1 
AGAAGGGGCTCCCGCACGCGCCTGCATTGATTAAGCGGACCAAC
AGAAGGATCCCCGAGAGGACATCACATCGAGTGGCAGGTTCCCA
ACTCGTGAAGAGTGAACTTGAGGAGAAAAGTCGGAGCTGCGGC
ACAAATTGAAATACGTACCGCATGAATACATCGAACTTATCGAAA
TTGCTAGGAACTCGACTCAAGACAGAATCCTTGAGATGAAGGTAA
TGGAGTTCTTTATGAAGGTTTATGGATACCGAGGGAAGCATCTCG
GTGGATCACGAAAACCCGACGGAGCAATCTATACGGTGGGAGC
CCGATTGATTACGGAGTGATCGTCGACACGAAAGCCTACAGCGG
TGGGTACAATCTTCCCATCGGGCAGGCAGATGAGATGCAACGTT
 
ATGTCGAAGAAAATCAGACCAGGAACAAACACATCAATCCAAATG
AGTGGTGGAAAGTGTATCCTTCATCAGTGACCGAGTTTAAGTTTT
TGTTTGTCTCTGGGCATTTCAAAGGCAACTATAAGGCCCAGCTCA
CACGGTTGAATCACATTACGAACTGCAATGGTGCGGTTTTGTCCG
TAGAGGAACTGCTCATTGGTGGAGAAATGATCAAAGCGGGAACT
CTGACACTGGAAGAAGTCAGACGCAAGTTTAACAATGGCGAGAT
CAATTTCCGCTCATAAAAATCAGCCTCGACTGTGCCTTCTAGTT
GCCAGCCATCTGTTGTTTGCCCCTCCCCCGTGCCTTCCTTGACC
CTGGAAGGTGCCACTCCCACTGTCCTTTCCTAATAAAATGAGGAA
ATTGCATCACAACACTCAACCCTATCTCGGTCTATTCTTTTGATTT
ATAAGGGATTTTGCCGATTTCGGCCTATTGGTTAAAAAATGAGCT
GATTTAACAAAAATTTAACGCGAATTAATTCTGTGGAATGTGTGTC
AGTTAGGGTGTGGAAAGTCCCCAGGCTCCCCAGCAGGCAGAAGT
ATGCAAAGCATGCATCTCAATTAGTCAGCAACCAGGTGTGGAAAG
TCCCCAGGCTCCCCAGCAGGCAGAAGTATGCAAAGCATGCATCT
CAATTAGTCAGCAACCATAGTCCCGCCCCTAACTCCGCCCATCC
CGCCCCTAACTCCGCCCAGTTCCGCCCATTCTCCGCCCCATGGC
TGACTAATTTTTTTTATTTATGCAGAGGCCGAGGCCGCCTCTGCC
TCTGAGCTATTCCAGAAGTAGTGAGGAGGCTTTTTTGGAGGCCTA
GGCTTTTGCAAAAAGCTCCCGGGAGCTTGTATATCCATTTTCGGA
TCTGATCAGCACGTGATGAAAAAGCCTGAACTCACCGCGACGTC
TGTCGAGAAGTTTCTGATCGAAAAGTTCGACAGCGTTTCCGACCT
GATGCAGCTCTCGGAGGGCGAAGAATCTCGTGCTTTCAGCTTCG
ATGTAGGAGGGCGTGGATATGTCCTGCGGGTAAATAGCTGCGCC
GATGGTTTCTACAAAGATCGTTATGTTTATCGGCACTTTGCATCG
 
GCCGCGCTCCCGATTCCGGAAGTGCTTGACATTGGGGAATTCAG
CGAGAGCCTGACCTATTGCATCTCCCGCCGTGCACAGGGTGTCA
CGTTGCAAGACCTGCCTGAAACCGAACTGCCCGCTGTTCTGCAG
CCGGTCGCGGAGGCCATGGATGCGATCGCTGCGGCCGATCTTA
GCCAGACGAGCGGGTTCGGCCCATTCGGACCGCAAGGAATCGG
TCAATACACTACATGGCGTGATTTCATATGCGCGATTGCTGATCC
CCATGTGTATCACTGGCAAACTGTGATGGACGACACCGTCAGTG
CGTCCGTCGCGCAGGCTCTCGATGAGCTGATGCTTTGGGCCGA
GGACTGCCCCGAAGTCCGGCACCTCGTGCACGCGGATTTCGGC
TCCAACAATGTCCTGACGGACAATGGCCGCATAACAGCGGTCAT
TGACTGGAGCGAGGCGATGTTCGGGGATTCCCAATACGAGGTCG
CCAACATCTTCTTCTGGAGGCCGTGGTTGGCTTGTATGGAGCAG
CAGACGCGCTACTTCGAGCGGAGGCATCCGGAGCTTGCAGGAT
CGCCGCGGCTCCGGGCGTATATGCTCCGCATTGGTCTTGACCAA
CTCTATCAGAGCTTGGTTGACGGCAATTTCGATGATGCAGCTTGG
GCGCAGGGTCGATGCGACGCAATCGTCCGATCCGGAGCCGGGA
CTGTCGGGCGTACACAAATCGCCCGCAGAAGCGCGGCCGTCTG
GACCGATGGCTGTGTAGAAGTACTCGCCGATAGTGGAAACCGAC
GCCCCAGCACTCGTCCGAGGGCAAAGGAATAGCACGTGCTACG
AGATTTCGATTCCACCGCCGCCTTCTATGAAAGGTTGGGCTTCG
GAATCGTTTTCCGGGACGCCGGCTGGATGATCCTCCAGCGCGG
GGATCTCATGCTGGAGTTCTTCGCCCACCCCAACTTGTTTATTGC
AGCTTATAATGGTTACAAATAAAGCAATAGCATCACAAATTTCACA
AATAAAGCATTTTTTTCACTGCATTCTAGTTGTGGTTTGTCCAAAC
TCATCAATGTATCTTATCATGTCTGTATACCGTCGACCTCTAGCTA
 
GAGCTTGGCGTAATCATGGTCATTACCAATGCTTAATCAGTGAGG
CACCTATCTCAGCGATCTGTCTATTTCGTTCATCCATAGTTGCCT
GACTCCCCGTCGTGTAGATAACTACGATACGGGAGGGCTTACCA
TCTGGCCCCAGCGCTGCGATGATACCGCGAGAACCACGCTCACC
GGCTCCGGATTTATCAGCAATAAACCAGCCAGCCGGAAGGGCCG
AGCGCAGAAGTGGTCCTGCAACTTTATCCGCCTCCATCCAGTCTA
TTAATTGTTGCCGGGAAGCTAGAGTAAGTAGTTCGCCAGTTAATA
GTTTGCGCAACGTTGTTGCCATCGCTACAGGCATCGTGGTGTCA
CGCTCGTCGTTTGGTATGGCTTCATTCAGCTCCGGTTCCCAACGA
TCAAGGCGAGTTACATGATCCCCCATGTTGTGCAAAAAAGCGGTT
AGCTCCTTCGGTCCTCCGATCGTTGTCAGAAGTAAGTTGGCCGC
AGTGTTATCACTCATGGTTATGGCAGCACTGCATAATTCTCTTACT
GTCATGCCATCCGTAAGATGCTTTTCTGTGACTGGTGAGTACTCA
ACCAAGTCATTCTGAGAATAGTGTATGCGGCGACCGAGTTGCTCT
TGCCCGGCGTCAATACGGGATAATACCGCGCCACATAGCAGAAC
TTTAAAAGTGCTCATCATTGGAAAACGTTCTTCGGGGCGAAAACT
CTCAAGGATCTTACCGCTGTTGAGATCCAGTTCGATGTAACCCAC
TCGTGCACCCAACTGATCTTCAGCATCTTTTACTTTCACCAGCGT
TTCTGGGTGAGCAAAAACAGGAAGGCAAAATGCCGCAAAAAAGG
GAATAAGGGCGACACGGAAATGTTGAATACTCATATTCTTCCTTT
TTCAATATTATTGAAGCATTTATCAGGGTTATTGTCTCATGAGCGG
ATACATATTTGAATGTATTTAGAAAAATAAACAAATAGGGGTCAGT
GTTACAACCAATTAACCAATTCTGAACATTATCGCGAGCCCATTTA
TACCTGAATATGGCTCATAACACCCCTTGCTCATGACCAAAATCC
CTTAACGTGAGTTACGCGCGCGTCGTTCCACTGAGCGTCAGACC
 
CCGTAGAAAAGATCAAAGGATCTTCTTGAGATCCTTTTTTTCTGC
GCGTAATCTGCTGCTTGCAAACAAAAAAACCACCGCTACCAGCG
GTGGTTTGTTTGCCGGATCAAGAGCTACCAACTCTTTTTCCGAAG
GTAACTGGCTTCAGCAGAGCGCAGATACCAAATACTGTTCTTCTA
GTGTAGCCGTAGTTAGCCCACCACTTCAAGAACTCTGTAGCACC
GCCTACATACCTCGCTCTGCTAATCCTGTTACCAGTGGCTGCTGC
CAGTGGCGATAAGTCGTGTCTTACCGGGTTGGACTCAAGACGAT
AGTTACCGGATAAGGCGCAGCGGTCGGGCTGAACGGGGGGTTC
GTGCACACAGCCCAGCTTGGAGCGAACGACCTACACCGAACTGA
GATACCTACAGCGTGAGCTATGAGAAAGCGCCACGCTTCCCGAA
GGGAGAAAGGCGGACAGGTATCCGGTAAGCGGCAGGGTCGGAA
CAGGAGAGCGCACGAGGGAGCTTCCAGGGGGAAACGCCTGGTA
TCTTTATAGTCCTGTCGGGTTTCGCCACCTCTGACTTGAGCGTCG
ATTTTTGTGATGCTCGTCAGGGGGGCGGAGCCTATGGAAAAACG
CCAGCAACGCGGCCTTTTTACGGTTCCTGGCCTTTTGCTGGCCTT
TTGCTCACATGTTCTTTCCTGCGTTATCCCCTGATTCTGTGGATAA
CCGTATTACCGCCTTTGAGTGAGCTGATACCGCTCGCCGCAGCC
GAACGACCGAGCGCAGCGAGTCAGTGAGCGAGGAAGCGGAAGG
CGAGAGTAGGGAACTGCCAGGCATCAAACTAAGCAGAAGGCCCC
TGACGGATGGCCTTTTTGCGTTTCTACAAACTCTTTCTGTGTTGTA
AAACGACGGCCAGTCTTAAGCTCGGGCCCCCTGGGCGGTTCTGA
TAACGAGTAATCGTTAATCCGCAAATAACGTAAAAACCCGCTTCG
GCGGGTTTTTTTATGGGGGGAGTTTAGGGAAAGAGCATTTGTCA
GAATATTTAAGGGCGCCTGTCACTTTGCTTGATATATGAGAATTAT
TTAACCTTATAAATGAGAAAAAAGCAACGCACTTTAAATAAGATAC
 
GTTGCTTTTTCGATTGATGAACACCTATAATTAAACTATTCATCTA
TTATTTATGATTTTTTGTATATACAATATTTCTAGTTTGTTAAAGAG
AATTAAGAAAATAAATCTCGAAAATAATAAAGGGAAAATCAGTTTT
TGATATCAAAATTATACATGTCAACGATAATACAAAATATAATACA
AACTATAAGATGTTATCAGTATTTATTATCATTTAGAATAAATTTTG
TGTCGCCCTTAATTGTGAGCGGATAACAATTACGAGCTTCATGCA
CAGTGGCGTTGACATTGATTATTGACTAGTTATTAATAGTAATCAA
TTACGGGGTCATTAGTTCATAGCCCATATATGGAGTTCCGCGTTA
CATAACTTACGGTAAATGGCCCGCCTGGCTGACCGCCCAACGAC
CCCCGCCCATTGACGTCAATAATGACGTATGTTCCCATAGTAACG
CCAATAGGGACTTTCCATTGACGTCAATGGGTGGAGTATTTACGG
TAAACTGCCCACTTGGCAGTACATCAAGTGTATCATATGCCAAGT
ACGCCCCCTATTGACGTCAATGACGGTAAATGGCCCGCCTGGCA
TTATGCCCAGTACATGACCTTATGGGACTTTCCTACTTGGCAGTA
CATCTACGTATTAGTCATCGCTATTACCATGGTGATGCGGTTTTG
GCAGTACATCAATGGGCGTGGATAGCGGTTTGACTCACGGGGAT
TTCCAAGTCTCCACCCCATTGACGTCAATGGGAGTTTGTTTTGGC
ACCAAAATCAACGGGACTTTCCAAAATGTCGTAACAACTCCGCCC
CATTGACGCAAATGGGCGGTAGGCGTGTACGGTGGGAGGTCTAT
ATAAGCAGAGCTCTCTGGCTAACTAGAGAACCCACTGCTTACTGG
CTTATCGAAATTAATACGACTCACTATAGGGAAGCTTCTTGTTCTT
TTTGCAGAAGCTCAGAATAAACGCTCAACTTTGGCCTCGAGGCCA
CCATGGACTATAAGGACCACGACGGAGACTACAAGGATCATGAT
ATTGATTACAAAGACGATGACGATAAGATGGCCCCAAAGAAGAAG
CGGAAGGTCGGTATCCACGGAGTCCCAGCAGCCGTAGATTTGAG
 
AACTTTGGGATATTCACAGCAGCAGCAGGAAAAGATCAAGCCCA
AAGTGAGGTCGACAGTCGCGCAGCATCACGAAGCGCTGGTGGG
TCATGGGTTTACACATGCCCACATCGTAGCCTTGTCGCAGCACC
CTGCAGCCCTTGGCACGGTCGCCGTCAAGTACCAGGACATGATT
GCGGCGTTGCCGGAAGCCACACATGAGGCGATCGTCGGTGTGG
GGAAACAGTGGAGCGGAGCCCGAGCGCTTGAGGCCCTGTTGAC
GGTCGCGGGAGAGCTGAGAGGGCCTCCCCTTCAGCTGGACACG
GGCCAGTTGCTGAAGATCGCGAAGCGGGGAGGAGTCACGGCGG
TCGAGGCGGTGCACGCGTGGCGCAATGCGCTCACGGGAGCACC
CCTCAACCTGACCCCAGACCAGGTTGTGGCCATCGCCAGCAACA
TAGGTGGCAAGCAGGCCCTCGAAACCGTCCAGAGACTGTTACCG
GTTCTCTGCCAGGACCACGGCCTGACCCCGGAACAGGTGGTTG
CAATCGCGTCACACGATGGGGGAAAGCAGGCCCTAGAAACCGTT
CAGCGACTCCTGCCCGTCCTGTGCCAGGCCCACGGCCTGACCC
CGGACCAGGTGGTTGCAATCGCGTCACACGATGGGGGAAAGCA
GGCCCTAGAAACCGTTCAGCGACTCCTGCCCGTCCTGTGCCAGG
CCCACGGCCTGACCCCCGCCCAGGTTGTCGCTATTGCTAGTAAC
GGCGGAGGCAAACAGGCGCTGGAAACAGTTCAGCGCCTCTTGC
CGGTCTTGTGTCAGGACCACGGCCTGACCCCAGACCAAGTTGTC
GCGATTGCAAGCAACAACGGAGGCAAACAAGCCTTAGAAACAGT
CCAGAGATTGTTGCCGGTGCTGTGCCAAGACCACGGCCTGACCC
CAGAACAAGTTGTCGCGATTGCAAGCAACAACGGAGGCAAACAA
GCCTTAGAAACAGTCCAGAGATTGTTGCCGGTGCTGTGCCAAGC
CCACGGCCTGACCCCAGACCAGGTTGTGGCCATCGCCAGCAAC
ATAGGTGGCAAGCAGGCCCTCGAAACCGTCCAGAGACTGTTACC
 
GGTTCTCTGCCAGGCCCACGGCCTGACCCCAGCCCAGGTTGTG
GCCATCGCCAGCAACATAGGTGGCAAGCAGGCCCTCGAAACCGT
CCAGAGACTGTTACCGGTTCTCTGCCAGGACCACGGCCTGACCC
CCGACCAGGTTGTCGCTATTGCTAGTAACGGCGGAGGCAAACAG
GCGCTGGAAACAGTTCAGCGCCTCTTGCCGGTCTTGTGTCAGGA
CCACGGCCTGACCCCCGAACAGGTTGTCGCTATTGCTAGTAACG
GCGGAGGCAAACAGGCGCTGGAAACAGTTCAGCGCCTCTTGCC
GGTCTTGTGTCAGGCCCACGGCCTGACCCCCGACCAGGTTGTC
GCTATTGCTAGTAACGGCGGAGGCAAACAGGCGCTGGAAACAGT
TCAGCGCCTCTTGCCGGTCTTGTGTCAGGCCCACGGCCTGACCC
CCGCCCAGGTTGTCGCTATTGCTAGTAACGGCGGAGGCAAACAG
GCGCTGGAAACAGTTCAGCGCCTCTTGCCGGTCTTGTGTCAGGA
CCACGGCCTGACCCCAGACCAAGTTGTCGCGATTGCAAGCAACA
ACGGAGGCAAACAAGCCTTAGAAACAGTCCAGAGATTGTTGCCG
GTGCTGTGCCAAGACCACGGCCTGACCCCCGAACAGGTTGTCG
CTATTGCTAGTAACGGCGGAGGCAAACAGGCGCTGGAAACAGTT
CAGCGCCTCTTGCCGGTCTTGTGTCAGGCCCACGGCCTGACCCC
GGACCAGGTGGTTGCAATCGCGTCACACGATGGGGGAAAGCAG
GCCCTAGAAACCGTTCAGCGACTCCTGCCCGTCCTGTGCCAGGC
CCACGGCCTGACCCCCGCCCAGGTTGTCGCTATTGCTAGTAACG
GCGGAGGCAAACAGGCGCTGGAAACAGTTCAGCGCCTCTTGCC
GGTCTTGTGTCAGGACCACGGCCTGACGCCTGAGCAGGTAGTG
GCTATTGCATCCAACGGAGGGGGCAGACCCGCACTGGAGTCAAT
CGTGGCCCAGCTTTCGAGGCCGGACCCCGCGCTGGCCGCACTC
ACTAATGATCATCTTGTAGCGCTGGCCTGCCTCGGCGGACGACC
 CGCCTTGGATGCGGTGA.
SEQ ID NO: 2

AGAAGGGGCTCCCGCACGCGCCTGCATTGATTAAGCGGACCAAC
AGAAGGATCCCCGAGAGGACATCACATCGAGTGGCAGGTTCCCA
ACTCGTGAAGAGTGAACTTGAGGAGAAAAGTCGGAGCTGCGGC
ACAAATTGAAATACGTACCGCATGAATACATCGAACTTATCGAAA
TTGCTAGGAACTCGACTCAAGACAGAATCCTTGAGATGAAGGTAA
TGGAGTTCTTTATGAAGGTTTATGGATACCGAGGGAAGCATCTCG
GTGGATCACGAAAACCCGACGGAGCAATCTATACGGTGGGGAGC
CCGATTGATTACGGAGTGATCGTCGACACGAAAGCCTACAGCGG
TGGGTACAATCTTCCCATCGGGCAGGCAGATGAGATGCAACGTT
ATGTCGAAGAAAATCAGACCAGGAACAAACACATCAATCCAAATG
AGTGGTGGAAAGTGTATCCTTCATCAGTGACCGAGTTTAAGTTTT
TGTTTGTCTCTGGGCATTTCAAAGGCAACTATAAGGCCCAGCTCA
CACGGTTGAATCACATTACGAACTGCAATGGTGCGGTTTTGTCCG
TAGAGGAACTGCTCATTGGTGGAGAAATGATCAAAGCGGGAACT
CTGACACTGGAAGAAGTCAGACGCAAGTTTAACAATGGCGAGAT
CAATTTCCGCTCATAAAAAATCAGCCTCGACTGTGCCTTCTAGTT
GCCAGCCATCTGTTGTTTGCCCCTCCCCCGTGCCTTCCTTGACC
CTGGAAGGTGCCACTCCCACTGTCCTTTCCTAATAAAATGAGGAA
ATTGCATCACAACACTCAACCCTATCTCGGTCTATTCTTTTGATTT
ATAAGGGATTTTGCCGATTTCGGCCTATTGGTTAAAAAATGAGCT
GATTTAACAAAAATTTAACGCGAATTAATTCTGTGGAATGTGTGTC
AGTTAGGGTGTGGAAAGTCCCCAGGCTCCCCAGCAGGCAGAAGT

ATGCAAAGCATGCATCTCAATTAGTCAGCAACCAGGTGTGGAAAG
TCCCCAGGCTCCCCAGCAGGCAGAAGTATGCAAAGCATGCATCT
CAATTAGTCAGCAACCATAGTCCCGCCCCTAACTCCGCCCATCC
CGCCCCTAACTCCGCCCAGTTCCGCCCATTCTCCGCCCCATGGC
TGACTAATTTTTTTTATTTATGCAGAGGCCGAGGCCGCCTCTGCC
TCTGAGCTATTCCAGAAGTAGTGAGGAGGCTTTTTTGGAGGCCTA
GGCTTTTGCAAAAAGCTCCCGGGAGCTTGTATATCCATTTTCGGA
TCTGATCAGCACGTGATGAAAAAGCCTGAACTCACCGCGACGTC
TGTCGAGAAGTTTCTGATCGAAAAGTTCGACAGCGTTTCCGACCT
GATGCAGCTCTCGGAGGGCGAAGAATCTCGTGCTTTCAGCTTCG
ATGTAGGAGGGCGTGGATATGTCCTGCGGGTAAATAGCTGCGCC
GATGGTTTCTACAAAGATCGTTATGTTTATCGGCACTTTGCATCG
GCCGCGCTCCCGATTCCGGAAGTGCTTGACATTGGGGAATTCAG
CGAGAGCCTGACCTATTGCATCTCCCGCCGTGCACAGGGTGTCA
CGTTGCAAGACCTGCCTGAAACCGAACTGCCCGCTGTTCTGCAG
CCGGTCGCGGAGGCCATGGATGCGATCGCTGCGGCCGATCTTA
GCCAGACGAGCGGGTTCGGCCCATTCGGACCGCAAGGAATCGG
TCAATACACTACATGGCGTGATTTCATATGCGCGATTGCTGATCC
CCATGTGTATCACTGGCAAACTGTGATGGACGACACCGTCAGTG
CGTCCGTCGCGCAGGCTCTCGATGAGCTGATGCTTTGGGCCGA
GGACTGCCCCGAAGTCCGGCACCTCGTGCACGCGGATTTCGGC
TCCAACAATGTCCTGACGGACAATGGCCGCATAACAGCGGTCAT
TGACTGGAGCGAGGCGATGTTCGGGGATTCCCAATACGAGGTCG
CCAACATCTTCTTCTGGAGGCCGTGGTTGGCTTGTATGGAGCAG
CAGACGCGCTACTTCGAGCGGAGGCATCCGGAGCTTGCAGGAT

CGCCGCGGCTCCGGGCGTATATGCTCCGCATTGGTCTTGACCAA
CTCTATCAGAGCTTGGTTGACGGCAATTTCGATGATGCAGCTTGG
GCGCAGGGTCGATGCGACGCAATCGTCCGATCCGGAGCCGGGA
CTGTCGGGCGTACACAAATCGCCCGCAGAAGCGCGGCCGTCTG
GACCGATGGCTGTGTAGAAGTACTCGCCGATAGTGGAAACCGAC
GCCCCAGCACTCGTCCGAGGGCAAAGGAATAGCACGTGCTACG
AGATTTCGATTCCACCGCCGCCTTCTATGAAAGGTTGGGCTTCG
GAATCGTTTTCCGGGACGCCGGCTGGATGATCCTCCAGCGCGG
GGATCTCATGCTGGAGTTCTTCGCCCACCCCAACTTGTTTATTGC
AGCTTATAATGGTTACAAATAAAGCAATAGCATCACAAATTTCACA
AATAAAGCATTTTTTTCACTGCATTCTAGTTGTGGTTTGTCCAAAC
TCATCAATGTATCTTATCATGTCTGTATACCGTCGACCTCTAGCTA
GAGCTTGGCGTAATCATGGTCATTACCAATGCTTAATCAGTGAGG
CACCTATCTCAGCGATCTGTCTATTTCGTTCATCCATAGTTGCCT
GACTCCCCGTCGTGTAGATAACTACGATACGGGAGGGCTTACCA
TCTGGCCCCAGCGCTGCGATGATACCGCGAGAACCACGCTCACC
GGCTCCGGATTTATCAGCAATAAACCAGCCAGCCGGAAGGGCCG
AGCGCAGAAGTGGTCCTGCAACTTTATCCGCCTCCATCCAGTCTA
TTAATTGTTGCCGGGAAGCTAGAGTAAGTAGTTCGCCAGTTAATA
GTTTGCGCAACGTTGTTGCCATCGCTACAGGCATCGTGGTGTCA
CGCTCGTCGTTTGGTATGGCTTCATTCAGCTCCGGTTCCCAACGA
TCAAGGCGAGTTACATGATCCCCCATGTTGTGCAAAAAAGCGGTT
AGCTCCTTCGGTCCTCCGATCGTTGTCAGAAGTAAGTTGGCCGC
AGTGTTATCACTCATGGTTATGGCAGCACTGCATAATTCTCTTACT
GTCATGCCATCCGTAAGATGCTTTTCTGTGACTGGTGAGTACTCA

ACCAAGTCATTCTGAGAATAGTGTATGCGGCGACCGAGTTGCTCT
TGCCCGGCGTCAATACGGGATAATACCGCGCCACATAGCAGAAC
TTTAAAAGTGCTCATCATTGGAAAACGTTCTTCGGGGCGAAAACT
CTCAAGGATCTTACCGCTGTTGAGATCCAGTTCGATGTAACCCAC
TCGTGCACCCAACTGATCTTCAGCATCTTTTACTTTCACCAGCGT
TTCTGGGTGAGCAAAAACAGGAAGGCAAAATGCCGCAAAAAAGG
GAATAAGGGCGACACGGAAATGTTGAATACTCATATTCTTCCTTT
TTCAATATTATTGAAGCATTTATCAGGGTTATTGTCTCATGAGCGG
ATACATATTTGAATGTATTTAGAAAAATAAACAAATAGGGGTCAGT
GTTACAACCAATTAACCAATTCTGAACATTATCGCGAGCCCATTTA
TACCTGAATATGGCTCATAACACCCCTTGCTCATGACCAAAATCC
CTTAACGTGAGTTACGCGCGTCGTTCCACTGAGCGTCAGACC
CCGTAGAAAAGATCAAAGGATCTTCTTGAGATCCTTTTTTTCTGC
GCGTAATCTGCTGCTTGCAAACAAAAAAACCACCGCTACCAGCG
GTGGTTTGTTTGCCGGATCAAGAGCTACCAACTCTTTTTCCGAAG
GTAACTGGCTTCAGCAGAGCGCAGATACCAAATACTGTTCTTCTA
GTGTAGCCGTAGTTAGCCCACCACTTCAAGAACTCTGTAGCACC
GCCTACATACCTCGCTCTGCTAATCCTGTTACCAGTGGCTGCTGC
CAGTGGCGATAAGTCGTGTCTTACCGGGTTGGACTCAAGACGAT
AGTTACCGGATAAGGCGCAGCGGTCGGGCTGAACGGGGGGTTC
GTGCACACAGCCCAGCTTGGAGCGAACGACCTACACCGAACTGA
GATACCTACAGCGTGAGCTATGAGAAAGCGCCACGCTTCCCGAA
GGGAGAAAGGCGGACAGGTATCCGGTAAGCGGCAGGGTCGGAA
CAGGAGAGCGCACGAGGGAGCTTCCAGGGGGAAACGCCTGGTA
TCTTTATAGTCCTGTCGGGTTTCGCCACCTCTGACTTGAGCGTCG

ATTTTTGTGATGCTCGTCAGGGGGGCGGAGCCTATGGAAAAACG
CCAGCAACGCGGCCTTTTTACGGTTCCTGGCCTTTTGCTGGCCTT
TTGCTCACATGTTCTTTCCTGCGTTATCCCCTGATTCTGTGGATAA
CCGTATTACCGCCTTTGAGTGAGCTGATACCGCTCGCCGCAGCC
GAACGACCGAGCGCAGCGAGTCAGTGAGCGAGGAAGCGGAAGG
CGAGAGTAGGGAACTGCCAGGCATCAAACTAAGCAGAAGGCCCC
TGACGGATGGCCTTTTTGCGTTTCTACAAACTCTTTCTGTGTTGTA
AAACGACGGCCAGTCTTAAGCTCGGGCCCCCTGGGCGGTTCTGA
TAACGAGTAATCGTTAATCCGCAAATAACGTAAAAACCCGCTTCG
GCGGGTTTTTTTATGGGGGGAGTTTAGGGAAAGAGCATTTGTCA
GAATATTTAAGGGCGCCTGTCACTTTGCTTGATATATGAGAATTAT
TTAACCTTATAAATGAGAAAAAAGCAACGCACTTTAAATAAGATAC
GTTGCTTTTTCGATTGATGAACACCTATAATTAAACTATTCATCTA
TTATTTATGATTTTTTGTATATACAATATTTCTAGTTTGTTAAAGAG
AATTAAGAAAATAAATCTCGAAAATAATAAAGGGAAAATCAGTTTT
TGATATCAAAATTATACATGTCAACGATAATACAAAATATAATACA
AACTATAAGATGTTATCAGTATTTATTATCATTTAGAATAAATTTTG
TGTCGCCCTTAATTGTGAGCGGATAACAATTACGAGCTTCATGCA
CAGTGGCGTTGACATTGATTATTGACTAGTTATTAATAGTAATCAA
TTACGGGGTCATTAGTTCATAGCCCATATATGGAGTTCCGCGTTA
CATAACTTACGGTAAATGGCCCGCCTGGCTGACCGCCCAACGAC
CCCCGCCCATTGACGTCAATAATGACGTATGTTCCCATAGTAACG
CCAATAGGGACTTTCCATTGACGTCAATGGGTGGAGTATTTACGG
TAAACTGCCCACTTGGCAGTACATCAAGTGTATCATATGCCAAGT
ACGCCCCCTATTGACGTCAATGACGGTAAATGGCCCGCCTGGCA

TTATGCCCAGTACATGACCTTATGGGACTTTCCTACTTGGCAGTA
CATCTACGTATTAGTCATCGCTATTACCATGGTGATGCGGTTTTG
GCAGTACATCAATGGGCGTGGATAGCGGTTTGACTCACGGGGAT
TTCCAAGTCTCCACCCCATTGACGTCAATGGGAGTTTGTTTTGGC
ACCAAAATCAACGGGACTTTCCAAAATGTCGTAACAACTCCGCCC
CATTGACGCAAATGGGCGGTAGGCGTGTACGGTGGGAGGTCTAT
ATAAGCAGAGCTCTCTGGCTAACTAGAGAACCCACTGCTTACTGG
CTTATCGAAATTAATACGACTCACTATAGGGAAGCTTCTTGTTCTT
TTTGCAGAAGCTCAGAATAAACGCTCAACTTTGGCCTCGAGGCCA
CCATGGACTATAAGGACCACGACGGAGACTACAAGGATCATGAT
ATTGATTACAAAGACGATGACGATAAGATGGCCCCAAAGAAGAAG
CGGAAGGTCGGTATCCACGGAGTCCCAGCAGCCGTAGATTTGAG
AACTTTGGGATATTCACAGCAGCAGCAGGAAAAGATCAAGCCCA
AAGTGAGGTCGACAGTCGCGCAGCATCACGAAGCGCTGGTGGG
TCATGGGTTTACACATGCCCACATCGTAGCCTTGTCGCAGCACC
CTGCAGCCCTTGGCACGGTCGCCGTCAAGTACCAGGACATGATT
GCGGCGTTGCCGGAAGCCACACATGAGGCGATCGTCGGTGTGG
GGAAACAGTGGAGCGGAGCCCGAGCGCTTGAGGCCCTGTTGAC
GGTCGCGGGAGAGCTGAGAGGGCCTCCCCTTCAGCTGGACACG
GGCCAGTTGCTGAAGATCGCGAAGCGGGGAGGAGTCACGGCGG
TCGAGGCGGTGCACGCGTGGCACAATGCGCTCACGGGAGCACC
CCTCAACCTGACCCCAGACCAGGTTGTGGCCATCGCCAGCAACA
TAGGTGGCAAGCAGGCCCTCGAAACCGTCCAGAGACTGTTACCG
GTTCTCTGCCAGGACCACGGCCTGACCCCCGAACAGGTTGTCGC
TATTGCTAGTAACGGCGGAGGCAAACAGGCGCTGGAAACAGTTC

AGCGCCTCTTGCCGGTCTTGTGTCAGGCCCACGGCCTGACCCCC
GACCAGGTTGTCGCTATTGCTAGTAACGGCGGAGGCAAACAGGC
GCTGGAAACAGTTCAGCGCCTCTTGCCGGTCTTGTGTCAGGCCC
ACGGCCTGACCCCGGCCCAGGTGGTTGCAATCGCGTCACACGA
TGGGGGAAAGCAGGCCCTAGAAACCGTTCAGCGACTCCTGCCC
GTCCTGTGCCAGGACCACGGCCTGACCCCAGACCAGGTTGTGG
CCATCGCCAGCAACATAGGTGGCAAGCAGGCCCTCGAAACCGTC
CAGAGACTGTTACCGGTTCTCTGCCAGGACCACGGCCTGACCCC
GGAACAGGTGGTTGCAATCGCGTCACACGATGGGGGAAAGCAG
GCCCTAGAAACCGTTCAGCGACTCCTGCCCGTCCTGTGCCAGGC
CCACGGCCTGACCCCGGACCAGGTGGTTGCAATCGCGTCACAC
GATGGGGGAAAGCAGGCCCTAGAAACCGTTCAGCGACTCCTGC
CCGTCCTGTGCCAGGCCCACGGCCTGACCCCAGCCCAGGTTGT
GGCCATCGCCAGCAACATAGGTGGCAAGCAGGCCCTCGAAACC
GTCCAGAGACTGTTACCGGTTCTCTGCCAGGACCACGGCCTGAC
CCCAGACCAGGTTGTGGCCATCGCCAGCAACATAGGTGGCAAGC
AGGCCCTCGAAACCGTCCAGAGACTGTTACCGGTTCTCTGCCAG
GACCACGGCCTGACCCCGGAACAGGTGGTTGCAATCGCGTCAC
ACGATGGGGGAAAGCAGGCCCTAGAAACCGTTCAGCGACTCCTG
CCCGTCCTGTGCCAGGCCCACGGCCTGACCCCGGACCAGGTGG
TTGCAATCGCGTCACACGATGGGGGAAAGCAGGCCCTAGAAACC
GTTCAGCGACTCCTGCCCGTCCTGTGCCAGGCCCACGGCCTGA
CCCCAGCCCAGGTTGTGGCCATCGCCAGCAACATAGGTGGCAA
GCAGGCCCTCGAAACCGTCCAGAGACTGTTACCGGTTCTCTGCC
AGGACCACGGCCTGACCCCGGACCAGGTGGTTGCAATCGCGTC

ACACGATGGGGGAAAGCAGGCCCTAGAAACCGTTCAGCGACTCC
TGCCCGTCCTGTGCCAGGACCACGGCCTGACCCCAGAACAGGTT
GTGGCCATCGCCAGCAACATAGGTGGCAAGCAGGCCCTCGAAA
CCGTCCAGAGACTGTTACCGGTTCTCTGCCAGGCCCACGGCCTG
ACCCCGGACCAGGTGGTTGCAATCGCGTCACACGATGGGGGAA
AGCAGGCCCTAGAAACCGTTCAGCGACTCCTGCCCGTCCTGTGC
CAGGCCCACGGCCTGACCCCGGCCCAGGTGGTTGCAATCGCGT
CACACGATGGGGGAAAGCAGGCCCTAGAAACCGTTCAGCGACTC
CTGCCCGTCCTGTGCCAGGACCACGGCCTGACGCCTGAGCAGG
TAGTGGCTATTGCATCCAACATAGGGGGCAGACCCGCACTGGAG
TCAATCGTGGCCCAGCTTTCGAGGCCGGACCCCGCGCTGGCCG
CACTCACTAATGATCATCTTGTAGCGCTGGCCTGCCTCGGCGGA
CGACCCGCCTTGGATGCGGTGA.

### In vitro maturation and in vitro fertilization

The TALEN injected ova were incubated for 24 hours or more in Porcine Oocyte Medium (Research Institute for the Functional Peptides, IFP1010P) containing inactivated FBS (final concentration: 5%), hFSH (Fuji Pharma, final concentration: 0.15 IU/mL), and hCG (ASKA Pharmaceutical, final concentration: 10 IU/mL). Then, the ova matured to MI (Metaphase I) or more were collected. Semen used for fertilization was collected from wild-type male marmosets, and *in vitro* fertilization (IVF) was carried out with use of TYH medium (LSI medience, DR01031). The next day (approximately 16 hours after the start of IVF), IVF was released. It was determined whether the fertilization was succeeded or not in accordance with the presence or absence of a prokaryotic cell. The fertilized ova thus obtained were collected.

### Embryo transfer and pregnancy management

The fertilized ova were subjected to early-stage *in vitro* incubation in Sequencial Cleav (Origio, 83040010A). Two or three days after the start of the incubation, the embryos grown to a 4-cell stage or higher were collected. The embryos thus collected were subjected to later-stage *in vitro* incubation in a medium that was Sequencial Blast (Origio, 83060010A) containing inactivated FBS (final concentration: 10%) and L-glutamine (final concentration: 2 mM). One week after the injection of TALEN, the embryos were transferred into the uteri of surrogate mothers through a noninvasive catheter operation. After the transfer of the embryos, the surrogate mothers were measured for a P4-value in the blood and undergone uterus observation by ultrasonic diagnostic equipment so as to determine pregnancy. Individuals confirmed to be pregnant undergone a periodical examination once a week so as to observe a fetal heart rate, and the presence or absence of a morphological abnormality, etc. A method for obtaining newborns was based on natural delivery. If natural delivery was judged to be difficult from the state(s) of the surrogate mother and/or fetus, a caesarean section was performed to obtain a newborn.

### Genomic DNA analysis

Genomic DNA was extracted, with use of QIAamp DNA Micro Kit (Qiagen, 56304), from hair of a newborn or cord blood obtained at the caesarean section. PCR for analyzing the *PSEN1* gene was carried out with use of KOD plus neo (TOYOBO, 401) and two primers, Psen1_Ex9_up1 (ACCCGCGACTCCCTATTATT: SEQ ID NO: 3) and Psen1_Ex9_dn1 (TGCCTTGACTGTATTGTTGG: SEQ ID NO: 4). The reaction was carried out under the following reaction condition: After heating at 94°C for 2 minutes, 35 cycles of 98°C for 10 seconds, 60°C for 10 seconds, and 68°C for 30 seconds were carried out. After the reaction, some of the resultants were subjected to electrophoresis on an agarose gel to check for amplification. Some of the amplification products were subjected to surveyor assay, with which genetic modification can be visually detected. The remaining ones were cloned to a sequence vector with use of Zero Blunt PCR Cloning Kit (Thermo fisher scientific, K275040), and the plasmids were introduced into *Escherichia coli* (strain DH5α) for transformation. From the resulting subclones, plasmids were collected and subjected to sequence analysis.

### cDNA analysis

Total RNA was extracted, with use of Nucleospin RNA plus XS (Takara, U0990B), from hair of a newborn or cord blood obtained at the caesarean section. Then, cDNA was synthesized with use of ReverTra Ace -α- (TOYOBO, FSK-101). PCR for checking for the deficiency of exon 9 in the *PSEN1* gene was carried out with use of KOD plus neo and a set of two primers designed on an exon in the vicinity of the deficient site of exon 9. The set of two primers was either of (i) Pn1_on_Ex7_up1 (TACCTCCCTGAATGGACTGC: SEQ ID NO: 5) and Pn1_on_Ex11_dn2 (TGGTTGTGTTCCAGTCTCCA: SEQ ID NO: 6) and (ii) Pn1_on_Ex8_up1 (GGTCCACTTCGTATGCTGGT: SEQ ID NO: 7) and Pn1_on_Ex11_dn1 (GGCTGTTGCTGAGGCTTTAC: SEQ ID NO: 8). The reaction was carried out under the following reaction condition: After heating at 94°C for 2 minutes, 35 cycles of 98°C for 10 seconds, 60°C for 10 seconds, and 68°C for 30 seconds were carried out. The samples undergone the reaction were subjected to electrophoresis on a 1.5% agarose gel. In the amplified products, band shifting was found to estimate the deficiency of exon 9. Some of the amplified products were cloned to a sequence vector with use of Zero Blunt PCR Cloning Kit, and the plasmids were introduced into *Escherichia coli* (strain DH5α) for transformation. From the resulting subclones, plasmids were collected and subjected to sequence analysis.

### Primary culture

A small amount of tissues were excised from ear lobes of newborns, and were cut into small pieces. The tissue pieces were put on a cell culture dish, and were cultured at 37°C in a 5% CO₂ environment with use of D-MEM (Thermo fisher scientific, 10566016) containing 10% inactivated FBS.

### Example 1

TALEN mRNAs targeting the 3'-splice site of exon 9 in the marmoset *PSEN1* gene were introduced into the nuclei of marmoset fertilized ova. Then, subcloning was carried out, followed by surveyor assay and sequencing assay. As shown in a of Fig. 2, the deficiency of the target site including the 3'-splice site sequence was found in two out of three fertilized ova, as expected. After the introduction of TALEN into the marmoset fertilized ova, RT-PCR and cDNA sequencing were carried out. Consequently, exclusion of exon 9 in the *PSEN1* mRNA was verified. As shown in b of Fig. 2, after the introduction of TALEN, an RNA was extracted from a 4-cell-stage fertilized ovum or a single blastomere. As shown in c of Fig. 2, complete exclusion of exon 9 was found in two 4-cell-stage fertilized ova and two single blastomeres. There was no wild-type sequence in the fertilized ova and single blastomeres in which the 3'-splice site was deficient. From this, it is considered that the 3'-splice site was made deficient in a biallelic manner. PS1 protein is a catalytic subunit for a γ-secretase complex having substrates that are type 1 transmembrane proteins including amyloid precursor protein (APP) and Notch. A region encoded by exon 9 exits in a site for endoproteolysis, and cleavage at this site is necessary for maturation of γ-secretase. Thus, exclusion of exon 9 leads to loss of the function of γ-secretase. The homozygous deletion of exon 9 in marmoset *PSEN1* causes embryonic lethality due to destruction of Notch signaling.

### Example 2

On the basis of the results obtained from the fertilized ova, an attempt was made to introduce TALEN mRNAs into ova in the above-described manner and to fertilize the ova. After fertilization, the fertilized ova were developed to the morula stage, and were then transferred to surrogate mothers. The introduction of TALEN did not cause any distinct adverse reaction in pregnancy or following development. In this manner, F0 marmosets (*PSEN1-*ΔE9) carrying a 6-bp deletion including the 3'-splice site in a heterozygous state could be obtained (a of Fig. 3 shows a photograph of a neonate). Genomic DNA extracted from the cord blood was subjected to surveyor assay. A result thereof is shown in b of Fig. 3. In b of Fig. 3, two left columns indicate results of the surveyor assay (Lane 1: a mixture of the genomic DNA of the neonate shown in a of Fig. 3 and a wild-type control blood (CB) sample, Lane 2: only the neonate genomic DNA) carried out by using, as a template, the neonate genomic DNA (the genomic DNA extracted from the cord blood of the neonate shown in a of Fig. 3). In b of Fig. 3, two right columns indicate controls for the experiment ("+" is a positive control, and "-" is a negative control). c of Fig. 3 shows results of PCR carried out using RNAs extracted from hair roots of the obtained mutant marmoset and the wild-type marmoset. In c of Fig. 3, two right columns indicate negative PCR data obtained from reactions of the same RNA samples taken place without RT. As indicated in c of Fig. 3, the PCR product with exclusion of exon 9 appeared as a 315 bp band, whereas the wild-type appeared as a 402 bp band. Note that the results obtained from the analyzed fertilized ova each indicated only a single band. From this, it is considered that a heterozygous mutation did not take place.

### Example 3

ELISA (Wako, 294-62501, 290-62601) was used to quantify Aβ in culture media of primary skin fibroblasts of a wild-type marmoset and *PSEN1-*ΔE9 (I774gmF) marmoset. A result thereof is shown in Fig. 4. In the fibroblasts of the *PSEN1-*ΔE9 marmoset, a decrease of Aβ₄₀, an increase of Aβ₄₂, and an increase of Aβ₄₂/Aβ₄₀ ratio were found. This shows that *PSEN1-*ΔE9 mutation has pathogenicity (i.e., *PSEN1-*ΔE9 mutation causes a disease).

Genomic DNA analysis and cDNA analysis indicated that, in two individuals with a mutation in the *PSEN1* gene, normal *PSEN1* and exon 9-deficient *PSEN1* appeared at a ratio of approximately 1:1. This strongly suggests that heterozygous state of *PSEN1-*ΔE9*,* which is found in human patients, could be replicated. In addition, according to the result of Aβ quantification in the fibroblast culture media, replication of an abnormality in Aβ production profile was found. From this, it is considered that the produced *PSEN1-*ΔE9 marmosets replicated a disease state of AD. The method, adopted in the Examples, for introducing a mutation in the 3'-splice site to induce exon skipping can be carried out by inducing DNA double strand break at a single site on a genomic DNA, and thus is considered to be more efficient and useful than a generally-conducted method for deleting an exon by cleaving both ends of the exon by genome editing and a knock-in method. Such a method for introducing, by genome editing, a mutation into a sequence related to splicing to produce a disease model that replicates a state of a disease (Alzheimer's disease) is considered to be useful. Furthermore, in a case where, as in the present invention, a targeted gene mutation can cause embryonic lethality if the gene mutation takes place in a homozygous status, a method for carrying out genome editing on ova and fertilizing the ova is considered to be useful for avoiding embryonic lethality.

### Example 4

In the same manner as that in Example 2, two *PSEN1-*ΔE9 neonate individuals (I943gmM and I949gmM) were further obtained (a of Fig. 5). For three individuals including I774gmF in addition to the two *PSEN1-*ΔE9 neonate individuals, surveyor assay was carried out on genomic DNAs extracted from cord blood. A result thereof is shown in b of Fig. 5. The meanings of the letters indicated in a lower part of b of Fig. 5 are as follows. "CB" denotes a genome extracted from cord blood of a neonate. "HR" denotes a genome extracted from hair roots of the neonate. "M" denotes a marker. "+N" indicates addition of Normal Genome. "+" and "-" indicate reagent controls. As indicated in c of Fig. 5, I774gmF and I943gmM had the *PSEN1* gene carrying a 6-bp deletion including the 3'-splice site in a heterozygous state, whereas I949gmM had the *PSEN1* gene carrying a 9-bp deletion including the 3'-splice site in a heterozygous state.

Western blot analysis was carried out against PS1 protein in membrane fractions obtained from the fibroblasts of the wild-type (I774gmF) and *PSEN1-*ΔE9 (I943gmM and I949gmM) marmosets. A result thereof is shown in a of Fig. 6. A left panel shows a result of the analysis involving use of an antibody to an N-terminal fragment (NTF) of the PS1 protein. A right panel shows a result of the analysis involving use of an antibody to a C-terminal fragment (CTF) of the PS1 protein. The arrowheads indicate NTF and CTF produced by endoproteolysis of PS1, whereas the arrows indicate a full-length PS1 protein. Sodium-potassium ATPase was used as a loading control of the membrane fraction.

In the culture media of the primary skin fibroblasts, Aβ was quantified by sandwich ELISA (Wako, 294-62501, 290-62601). A result thereof is shown in c of Fig. 6. c of Fig. 6 indicates an average value ± a standard error (WT: n=15, ΔE9: n=9). In c of Fig. 6, "**P≤0.01" and "****P≤0.0001" each indicate the presence of a significant difference as a result of Student's two-tailed t-test. Similarly to Example 3, a decrease of Aβ₄₀, an increase of Aβ₄₂, and an increase of Aβ₄₂/Aβ₄₀ ratio were found. The Aβ₄₃ level was less than a detection limit, and therefore is not illustrated.

### Industrial Applicability

A non-human primate AD model in accordance with an aspect of the present invention is very useful for development of a method for diagnosis and therapy of Alzheimer's disease.

### References

1. Saito, T. et al. Single App knock-in mouse models of Alzheimer's disease. Nat Neurosci 17, 661-663, doi:10.1038/nn.3697 (2014).
2. Sasaguri, H. et al. APP mouse models for Alzheimer's disease preclinical studies. EMBO J 36, 2473-2487, doi:10.15252/embj.201797397 (2017).
3. Sasaki, E. et al. Generation of transgenic non-human primates with germline transmission. Nature 459, 523-527, doi:10.1038/nature08090 (2009).
4. Izpisua Belmonte, J. C. et al. Brains, genes, and primates. Neuron 86, 617-631, doi:10.1016/j.neuron.2015.03.021 (2015).
5. Miller, C. T. et al. Marmosets: A Neuroscientific Model of Human Social Behavior. Neuron 90, 219-233, doi:10.1016/j.neuron.2016.03.018 (2016).
6. Crook, R. et al. A variant of Alzheimer's disease with spastic paraparesis and unusual plaques due to deletion of exon 9 of presenilin 1. Nature medicine 4, 452-455 (1998).
7. Prihar, G. et al. Alzheimer disease PS-1 exon 9 deletion defined. Nature medicine 5, 1090, doi:10.1038/13383 (1999).
8. Smith, M. J. et al. Variable phenotype of Alzheimer's disease with spastic paraparesis. Annals of neurology 49, 125-129 (2001).
9. Le Guennec, K. et al. Deletion of exons 9 and 10 of the Presenilin 1 gene in a patient with Early-onset Alzheimer Disease generates longer amyloid seeds. Neurobiology of disease 104, 97-103, doi:10.1016/j.nbd.2017.04.020 (2017).
10. Blauwendraat, C. et al. Pilot whole-exome sequencing of a German early-onset Alzheimer's disease cohort reveals a substantial frequency of PSEN2 variants. Neurobiology of aging 37, 208.e211-208.e217, doi:10.1016/j.neurobiolaging.2015.09.016 (2016).
11. Hutton, M. et al. Complete analysis of the presenilin 1 gene in early onset Alzheimer's disease. Neuroreport 7, 801-805 (1996).
12. Kwok, J. B. et al. Two novel (M233T and R278T) presenilin-1 mutations in early-onset Alzheimer's disease pedigrees and preliminary evidence for association of presenilin-1 mutations with a novel phenotype. Neuroreport 8, 1537-1542 (1997).
13. Brooks, W. S. et al. Alzheimer's disease with spastic paraparesis and 'cotton wool' plaques: two pedigrees with PS-1 exon 9 deletions. Brain : a journal of neurology 126, 783-791 (2003).
14. Sato, K. et al. Generation of a Nonhuman Primate Model of Severe Combined Immunodeficiency Using Highly Efficient Genome Editing. Cell stem cell 19, 127-138, doi:10.1016/j.stem.2016.06.003 (2016).
15. Tomita, T. & Iwatsubo, T. Structural biology of presenilins and signal peptide peptidases. The Journal of biological chemistry 288, 14673-14680, doi:10.1074/jbc.R113.463281 (2013).
16. Shen, J. et al. Skeletal and CNS defects in Presenilin-1-deficient mice. Cell 89, 629-639 (1997).
17. Kumar-Singh, S. et al. Mean age-of-onset of familial Alzheimer disease caused by presenilin mutations correlates with both increased A beta 42 and decreased A beta 40. Human Mutation 27, 686-695, doi:10.1002/humu.20336 (2006).
18. Sakuma, T. et al. Repeating pattern of non-RVD variations in DNA-binding modules enhances TALEN activity. Scientific reports 3, 3379, doi:10.1038/srep03379 (2013).
19. Geula, C., Nagykery, N. & Wu, C. K. Amyloid-beta deposits in the cerebral cortex of the aged common marmoset (Callithrix jacchus): incidence and chemical composition. Acta neuropathologica 103, 48-58 (2002).
20. Ridley, R. M., Baker, H. F., Windle, C. P. & Cummings, R. M. Very long term studies of the seeding of beta-amyloidosis in primates. Journal of neural transmission (Vienna, Austria : 1996) 113, 1243-1251, doi:10.1007/s00702-005-0385-2 (2006).
21. Rodriguez-Callejas, J. D., Fuchs, E. & Perez-Cruz, C. Evidence of Tau Hyperphosphorylation and Dystrophic Microglia in the Common Marmoset. Frontiers in aging neuroscience 8, 315, doi:10.3389/fnagi.2016.00315 (2016).
22. t Hart, B. A. & Massacesi, L. Clinical, pathological, and immunologic aspects of the multiple sclerosis model in common marmosets (Callithrix jacchus). Journal of neuropathology and experimental neurology 68, 341-355, doi:10.1097/NEN.0b013e31819f1d24 (2009).
23. Tardif, S. D., Mansfield, K. G., Ratnam, R., Ross, C. N. & Ziegler, T. E. The marmoset as a model of aging and age-related diseases. ILAR journal 52, 54-65 (2011).
24. Crofts, H. S. et al. Investigation of the sleep electrocorticogram of the common marmoset (Callithrix jacchus) using radiotelemetry. Clinical neurophysiology : official journal of the International Federation of Clinical Neurophysiology 112, 2265-2273 (2001).
25. Hikishima, K. et al. Atlas of the developing brain of the marmoset monkey constructed using magnetic resonance histology. Neuroscience 230, 102-113, doi:10.1016/j.neuroscience.2012.09.053 (2013).
26. Senoo, A., Tokuno, H. & Watson, C. Mini-atlas of the marmoset brain. Neuroscience research 93, 128-135, doi:10.1016/j.neures.2014.12.014 (2015).
27. Hashikawa, T., Nakatomi, R. & Iriki, A. Current models of the marmoset brain. Neuroscience research 93, 116-127, doi:10.1016/j.neures.2015.01.009 (2015).

## Claims

1. A non-human primate model animal of Alzheimer's disease, comprising:
the *PSEN1* gene in which a site including at least a 5'- or 3'-splice site of exon 9 and being related to splicing of exon 9 is deficient.

2. The non-human primate model animal of Alzheimer's disease as set forth in claim 1, wherein
the non-human primate is a marmoset.

3. A method for producing a non-human primate model animal of Alzheimer's disease, the method comprising:
making a site of the *PSEN1* gene deficient by a genome-editing technology, the site including at least a 5'- or 3'-splice site of exon 9 in the *PSEN1* gene and being related to splicing of exon 9.

4. The method as set forth in claim 3, wherein
the site including at least the 5'- or 3'-splice site of exon 9 in the *PSEN1* gene and being related to splicing of exon 9 is made deficient in an ovum.

5. The method as set forth in claim 3 or 4, wherein the non-human primate is a marmoset.


SEQUENCE LISTING

<110>  RIKEN, Central Institute for Experimental Animals, and
       Hiroshima University

<120>  NON-HUMAN PRIMATES MODEL ANIMALS OF ALZHEIMER'S DISEASE, AND
       METHOD FOR PRODUCING SAME

<130>  RK19442PCT

<150>  US 62/951,498
<151>  2019-12-20

<160>  8

<170>  PatentIn version 3.5

<210>  1
<211>  8174
<212>  DNA
<213>  artificial sequence

<220>
<223>  vector sequence for Left TALEN

<400>  1
agaaggggct cccgcacgcg cctgcattga ttaagcggac caacagaagg atccccgaga      60

ggacatcaca tcgagtggca ggttcccaac tcgtgaagag tgaacttgag gagaaaaagt     120

cggagctgcg gcacaaattg aaatacgtac cgcatgaata catcgaactt atcgaaattg     180

ctaggaactc gactcaagac agaatccttg agatgaaggt aatggagttc tttatgaagg     240

tttatggata ccgagggaag catctcggtg gatcacgaaa acccgacgga gcaatctata     300

cggtggggag cccgattgat tacggagtga tcgtcgacac gaaagcctac agcggtgggt     360

acaatcttcc catcgggcag gcagatgaga tgcaacgtta tgtcgaagaa aatcagacca     420

ggaacaaaca catcaatcca aatgagtggt ggaaagtgta tccttcatca gtgaccgagt     480

ttaagttttt gtttgtctct gggcatttca aaggcaacta taaggcccag ctcacacggt     540

tgaatcacat tacgaactgc aatggtgcgg ttttgtccgt agaggaactg ctcattggtg     600

gagaaatgat caaagcggga actctgacac tggaagaagt cagacgcaag tttaacaatg     660

gcgagatcaa tttccgctca taaaaaatca gcctcgactg tgccttctag ttgccagcca     720

tctgttgttt gcccctcccc cgtgccttcc ttgaccctgg aaggtgccac tcccactgtc     780

ctttcctaat aaaatgagga aattgcatca caacactcaa ccctatctcg gtctattctt     840

ttgatttata agggattttg ccgatttcgg cctattggtt aaaaaatgag ctgatttaac     900

aaaaatttaa cgcgaattaa ttctgtggaa tgtgtgtcag ttagggtgtg gaaagtcccc     960

aggctcccca gcaggcagaa gtatgcaaag catgcatctc aattagtcag caaccaggtg    1020

tggaaagtcc ccaggctccc cagcaggcag aagtatgcaa agcatgcatc tcaattagtc    1080

agcaaccata gtcccgcccc taactccgcc catcccgccc ctaactccgc ccagttccgc    1140

ccattctccg ccccatggct gactaatttt ttttatttat gcagaggccg aggccgcctc 1200

tgcctctgag ctattccaga agtagtgagg aggctttttt ggaggcctag gcttttgcaa 1260

aaagctcccg ggagcttgta tatccatttt cggatctgat cagcacgtga tgaaaaagcc 1320

tgaactcacc gcgacgtctg tcgagaagtt tctgatcgaa aagttcgaca gcgtttccga 1380

cctgatgcag ctctcggagg gcgaagaatc tcgtgctttc agcttcgatg taggagggcg 1440

tggatatgtc ctgcgggtaa atagctgcgc cgatggtttc tacaaagatc gttatgttta 1500

tcggcacttt gcatcggccg cgctcccgat tccggaagtg cttgacattg gggaattcag 1560

cgagagcctg acctattgca tctcccgccg tgcacagggt gtcacgttgc aagacctgcc 1620

tgaaaccgaa ctgcccgctg ttctgcagcc ggtcgcggag gccatggatg cgatcgctgc 1680

ggccgatctt agccagacga gcgggttcgg cccattcgga ccgcaaggaa tcggtcaata 1740

cactacatgg cgtgatttca tatgcgcgat tgctgatccc catgtgtatc actggcaaac 1800

tgtgatggac gacaccgtca gtgcgtccgt cgcgcaggct ctcgatgagc tgatgctttg 1860

ggccgaggac tgccccgaag tccggcacct cgtgcacgcg gatttcggct ccaacaatgt 1920

cctgacggac aatggccgca taacagcggt cattgactgg agcgaggcga tgttcgggga 1980

ttcccaatac gaggtcgcca acatcttctt ctggaggccg tggttggctt gtatggagca 2040

gcagacgcgc tacttcgagc ggaggcatcc ggagcttgca ggatcgccgc ggctccgggc 2100

gtatatgctc cgcattggtc ttgaccaact ctatcagagc ttggttgacg gcaatttcga 2160

tgatgcagct tgggcgcagg gtcgatgcga cgcaatcgtc cgatccggag ccgggactgt 2220

cgggcgtaca caaatcgccc gcagaagcgc ggccgtctgg accgatggct gtgtagaagt 2280

actcgccgat agtggaaacc gacgccccag cactcgtccg agggcaaagg aatagcacgt 2340

gctacgagat ttcgattcca ccgccgcctt ctatgaaagg ttgggcttcg gaatcgtttt 2400

ccgggacgcc ggctggatga tcctccagcg cggggatctc atgctggagt tcttcgccca 2460

ccccaacttg tttattgcag cttataatgg ttacaaataa agcaatagca tcacaaattt 2520

cacaaataaa gcattttttt cactgcattc tagttgtggt ttgtccaaac tcatcaatgt 2580

atcttatcat gtctgtatac cgtcgacctc tagctagagc ttggcgtaat catggtcatt 2640

accaatgctt aatcagtgag gcacctatct cagcgatctg tctatttcgt tcatccatag 2700

ttgcctgact ccccgtcgtg tagataacta cgatacggga gggcttacca tctggcccca 2760

gcgctgcgat gataccgcga gaaccacgct caccggctcc ggatttatca gcaataaacc 2820

agccagccgg aagggccgag cgcagaagtg gtcctgcaac tttatccgcc tccatccagt 2880

ctattaattg ttgccgggaa gctagagtaa gtagttcgcc agttaatagt ttgcgcaacg 2940

ttgttgccat cgctacaggc atcgtggtgt cacgctcgtc gtttggtatg gcttcattca 3000

gctccggttc ccaacgatca aggcgagtta catgatcccc catgttgtgc aaaaaagcgg 3060

ttagctcctt cggtcctccg atcgttgtca gaagtaagtt ggccgcagtg ttatcactca 3120

tggttatggc agcactgcat aattctctta ctgtcatgcc atccgtaaga tgcttttctg 3180

tgactggtga gtactcaacc aagtcattct gagaatagtg tatgcggcga ccgagttgct 3240

cttgcccggc gtcaatacgg gataataccg cgccacatag cagaacttta aaagtgctca 3300

tcattggaaa acgttcttcg gggcgaaaac tctcaaggat cttaccgctg ttgagatcca 3360

gttcgatgta acccactcgt gcacccaact gatcttcagc atcttttact ttcaccagcg 3420

tttctgggtg agcaaaaaca ggaaggcaaa atgccgcaaa aaagggaata agggcgacac 3480

ggaaatgttg aatactcata ttcttccttt ttcaatatta ttgaagcatt tatcagggtt 3540

attgtctcat gagcggatac atatttgaat gtatttagaa aaataaacaa ataggggtca 3600

gtgttacaac caattaacca attctgaaca ttatcgcgag cccatttata cctgaatatg 3660

gctcataaca ccccttgctc atgaccaaaa tcccttaacg tgagttacgc gcgcgtcgtt 3720

ccactgagcg tcagaccccg tagaaaagat caaaggatct tcttgagatc ctttttttct 3780

gcgcgtaatc tgctgcttgc aaacaaaaaa accaccgcta ccagcggtgg tttgtttgcc 3840

ggatcaagag ctaccaactc tttttccgaa ggtaactggc ttcagcagag cgcagatacc 3900

aaatactgtt cttctagtgt agccgtagtt agcccaccac ttcaagaact ctgtagcacc 3960

gcctacatac ctcgctctgc taatcctgtt accagtggct gctgccagtg gcgataagtc 4020

gtgtcttacc gggttggact caagacgata gttaccggat aaggcgcagc ggtcgggctg 4080

aacggggggt tcgtgcacac agcccagctt ggagcgaacg acctacaccg aactgagata 4140

cctacagcgt gagctatgag aaagcgccac gcttcccgaa gggagaaagg cggacaggta 4200

tccggtaagc ggcagggtcg gaacaggaga gcgcacgagg gagcttccag ggggaaacgc 4260

ctggtatctt tatagtcctg tcgggtttcg ccacctctga cttgagcgtc gatttttgtg 4320

atgctcgtca ggggggcgga gcctatggaa aaacgccagc aacgcggcct ttttacggtt 4380

cctggccttt tgctggcctt ttgctcacat gttctttcct gcgttatccc ctgattctgt 4440

ggataaccgt attaccgcct ttgagtgagc tgataccgct cgccgcagcc gaacgaccga 4500

gcgcagcgag tcagtgagcg aggaagcgga aggcgagagt agggaactgc caggcatcaa 4560

actaagcaga aggcccctga cggatggcct ttttgcgttt ctacaaactc tttctgtgtt 4620

gtaaaacgac ggccagtctt aagctcgggc cccctgggcg gttctgataa cgagtaatcg 4680

ttaatccgca aataacgtaa aaacccgctt cggcgggttt ttttatgggg ggagtttagg 4740

gaaagagcat ttgtcagaat atttaagggc gcctgtcact ttgcttgata tatgagaatt 4800

atttaacctt ataaatgaga aaaaagcaac gcactttaaa taagatacgt tgctttttcg 4860

attgatgaac acctataatt aaactattca tctattattt atgatttttt gtatatacaa 4920

tatttctagt ttgttaaaga gaattaagaa aataaatctc gaaaataata aagggaaaat 4980

cagtttttga tatcaaaatt atacatgtca acgataatac aaaatataat acaaactata 5040

agatgttatc agtatttatt atcatttaga ataaattttg tgtcgccctt aattgtgagc 5100

ggataacaat tacgagcttc atgcacagtg gcgttgacat tgattattga ctagttatta 5160

atagtaatca attacggggt cattagttca tagcccatat atggagttcc gcgttacata 5220

acttacggta aatggcccgc ctggctgacc gcccaacgac ccccgcccat tgacgtcaat 5280

aatgacgtat gttcccatag taacgccaat agggactttc cattgacgtc aatgggtgga 5340

gtatttacgg taaactgccc acttggcagt acatcaagtg tatcatatgc caagtacgcc 5400

ccctattgac gtcaatgacg gtaaatggcc cgcctggcat tatgcccagt acatgacctt 5460

atgggacttt cctacttggc agtacatcta cgtattagtc atcgctatta ccatggtgat 5520

gcggttttgg cagtacatca atgggcgtgg atagcggttt gactcacggg gatttccaag 5580

tctccacccc attgacgtca atgggagttt gttttggcac caaaatcaac gggactttcc 5640

aaaatgtcgt aacaactccg ccccattgac gcaaatgggc ggtaggcgtg tacggtggga 5700

ggtctatata agcagagctc tctggctaac tagagaaccc actgcttact ggcttatcga 5760

aattaatacg actcactata gggaagcttc ttgttctttt tgcagaagct cagaataaac 5820

gctcaacttt ggcctcgagg ccaccatgga ctataaggac cacgacggag actacaagga 5880

tcatgatatt gattacaaag acgatgacga taagatggcc ccaaagaaga agcggaaggt 5940

cggtatccac ggagtcccag cagccgtaga tttgagaact ttgggatatt cacagcagca 6000

gcaggaaaag atcaagccca aagtgaggtc gacagtcgcg cagcatcacg aagcgctggt 6060

gggtcatggg tttacacatg cccacatcgt agccttgtcg cagcaccctg cagcccttgg 6120

cacggtcgcc gtcaagtacc aggacatgat tgcggcgttg ccggaagcca cacatgaggc 6180

gatcgtcggt gtggggaaac agtggagcgg agcccgagcg cttgaggccc tgttgacggt 6240

cgcgggagag ctgagagggc ctccccttca gctggacacg ggccagttgc tgaagatcgc 6300

gaagcgggga ggagtcacgg cggtcgaggc ggtgcacgcg tggcgcaatg cgctcacggg 6360

agcacccctc aacctgaccc cagaccaggt tgtggccatc gccagcaaca taggtggcaa 6420

gcaggccctc gaaaccgtcc agagactgtt accggttctc tgccaggacc acggcctgac 6480

cccggaacag gtggttgcaa tcgcgtcaca cgatggggga aagcaggccc tagaaaccgt 6540

tcagcgactc ctgcccgtcc tgtgccaggc ccacggcctg accccggacc aggtggttgc 6600

aatcgcgtca cacgatgggg gaaagcaggc cctagaaacc gttcagcgac tcctgcccgt 6660

cctgtgccag gcccacggcc tgacccccgc ccaggttgtc gctattgcta gtaacggcgg 6720

aggcaaacag gcgctggaaa cagttcagcg cctcttgccg gtcttgtgtc aggaccacgg 6780

cctgacccca gaccaagttg tcgcgattgc aagcaacaac ggaggcaaac aagccttaga 6840

aacagtccag agattgttgc cggtgctgtg ccaagaccac ggcctgaccc cagaacaagt 6900

tgtcgcgatt gcaagcaaca acggaggcaa acaagcctta gaaacagtcc agagattgtt 6960

gccggtgctg tgccaagccc acggcctgac cccagaccag gttgtggcca tcgccagcaa 7020

cataggtggc aagcaggccc tcgaaaccgt ccagagactg ttaccggttc tctgccaggc 7080

ccacggcctg accccagccc aggttgtggc catcgccagc aacataggtg gcaagcaggc 7140

cctcgaaacc gtccagagac tgttaccggt tctctgccag gaccacggcc tgacccccga 7200

ccaggttgtc gctattgcta gtaacggcgg aggcaaacag gcgctggaaa cagttcagcg 7260

cctcttgccg gtcttgtgtc aggaccacgg cctgaccccc gaacaggttg tcgctattgc 7320

tagtaacggc ggaggcaaac aggcgctgga aacagttcag cgcctcttgc cggtcttgtg 7380

tcaggcccac ggcctgaccc ccgaccaggt tgtcgctatt gctagtaacg gcggaggcaa 7440

acaggcgctg gaaacagttc agcgcctctt gccggtcttg tgtcaggccc acggcctgac 7500

ccccgcccag gttgtcgcta ttgctagtaa cggcggaggc aaacaggcgc tggaaacagt 7560

tcagcgcctc ttgccggtct tgtgtcagga ccacggcctg accccagacc aagttgtcgc 7620

gattgcaagc aacaacggag gcaaacaagc cttagaaaca gtccagagat tgttgccggt 7680

gctgtgccaa gaccacggcc tgacccccga acaggttgtc gctattgcta gtaacggcgg 7740

aggcaaacag gcgctggaaa cagttcagcg cctcttgccg gtcttgtgtc aggcccacgg 7800

cctgaccccg gaccaggtgg ttgcaatcgc gtcacacgat gggggaaagc aggccctaga 7860

aaccgttcag cgactcctgc ccgtcctgtg ccaggcccac ggcctgaccc ccgcccaggt 7920

tgtcgctatt gctagtaacg gcggaggcaa acaggcgctg gaaacagttc agcgcctctt 7980

gccggtcttg tgtcaggacc acggcctgac gcctgagcag gtagtggcta ttgcatccaa 8040

cggagggggc agacccgcac tggagtcaat cgtggcccag ctttcgaggc cggaccccgc 8100

gctggccgca ctcactaatg atcatcttgt agcgctggcc tgcctcggcg gacgacccgc 8160

cttggatgcg gtga 8174

<210> 2
<211> 8174
<212> DNA
<213> artificial sequence

<220>
<223> vector sequence for Right TALEN

<400> 2
agaaggggct cccgcacgcg cctgcattga ttaagcggac caacagaagg atccccgaga 60

ggacatcaca tcgagtggca ggttcccaac tcgtgaagag tgaacttgag gagaaaaagt 120

cggagctgcg gcacaaattg aaatacgtac cgcatgaata catcgaactt atcgaaattg 180

ctaggaactc gactcaagac agaatccttg agatgaaggt aatggagttc tttatgaagg 240

tttatggata ccgagggaag catctcggtg gatcacgaaa acccgacgga gcaatctata 300

cggtggggag cccgattgat tacggagtga tcgtcgacac gaaagcctac agcggtgggt 360

acaatcttcc catcgggcag gcagatgaga tgcaacgtta tgtcgaagaa aatcagacca 420

ggaacaaaca catcaatcca aatgagtggt ggaaagtgta tccttcatca gtgaccgagt 480

ttaagttttt gtttgtctct gggcatttca aaggcaacta taaggcccag ctcacacggt 540

tgaatcacat tacgaactgc aatggtgcgg ttttgtccgt agaggaactg ctcattggtg 600

gagaaatgat caaagcggga actctgacac tggaagaagt cagacgcaag tttaacaatg 660

gcgagatcaa tttccgctca taaaaaatca gcctcgactg tgccttctag ttgccagcca 720

tctgttgttt gcccctcccc cgtgccttcc ttgaccctgg aaggtgccac tcccactgtc 780

ctttcctaat aaaatgagga aattgcatca caacactcaa ccctatctcg gtctattctt 840

ttgatttata agggattttg ccgatttcgg cctattggtt aaaaaatgag ctgatttaac 900

aaaaatttaa cgcgaattaa ttctgtggaa tgtgtgtcag ttagggtgtg gaaagtcccc 960

aggctcccca gcaggcagaa gtatgcaaag catgcatctc aattagtcag caaccaggtg 1020

tggaaagtcc ccaggctccc cagcaggcag aagtatgcaa agcatgcatc tcaattagtc 1080

agcaaccata gtcccgcccc taactccgcc catcccgccc ctaactccgc ccagttccgc 1140

ccattctccg ccccatggct gactaatttt ttttatttat gcagaggccg aggccgcctc 1200

tgcctctgag ctattccaga agtagtgagg aggctttttt ggaggcctag gcttttgcaa 1260

aaagctcccg ggagcttgta tatccatttt cggatctgat cagcacgtga tgaaaaagcc 1320

tgaactcacc gcgacgtctg tcgagaagtt tctgatcgaa aagttcgaca gcgtttccga 1380

cctgatgcag ctctcggagg gcgaagaatc tcgtgctttc agcttcgatg taggagggcg 1440

tggatatgtc ctgcgggtaa atagctgcgc cgatggtttc tacaaagatc gttatgttta 1500

tcggcacttt gcatcggccg cgctcccgat tccggaagtg cttgacattg gggaattcag 1560

cgagagcctg acctattgca tctcccgccg tgcacagggt gtcacgttgc aagacctgcc 1620

tgaaaccgaa ctgcccgctg ttctgcagcc ggtcgcggag gccatggatg cgatcgctgc 1680

ggccgatctt agccagacga gcgggttcgg cccattcgga ccgcaaggaa tcggtcaata 1740

cactacatgg cgtgatttca tatgcgcgat tgctgatccc catgtgtatc actggcaaac 1800

tgtgatggac gacaccgtca gtgcgtccgt cgcgcaggct ctcgatgagc tgatgctttg 1860

ggccgaggac tgccccgaag tccggcacct cgtgcacgcg gatttcggct ccaacaatgt 1920

cctgacggac aatggccgca taacagcggt cattgactgg agcgaggcga tgttcgggga 1980

ttcccaatac gaggtcgcca acatcttctt ctggaggccg tggttggctt gtatggagca 2040

gcagacgcgc tacttcgagc ggaggcatcc ggagcttgca ggatcgccgc ggctccgggc 2100

gtatatgctc cgcattggtc ttgaccaact ctatcagagc ttggttgacg gcaatttcga 2160

tgatgcagct tgggcgcagg gtcgatgcga cgcaatcgtc cgatccggag ccgggactgt 2220

cgggcgtaca caaatcgccc gcagaagcgc ggccgtctgg accgatggct gtgtagaagt 2280

actcgccgat agtggaaacc gacgccccag cactcgtccg agggcaaagg aatagcacgt 2340

gctacgagat ttcgattcca ccgccgcctt ctatgaaagg ttgggcttcg gaatcgtttt 2400

ccgggacgcc ggctggatga tcctccagcg cggggatctc atgctggagt tcttcgccca 2460

ccccaacttg tttattgcag cttataatgg ttacaaataa agcaatagca tcacaaattt 2520

cacaaataaa gcattttttt cactgcattc tagttgtggt ttgtccaaac tcatcaatgt 2580

atcttatcat gtctgtatac cgtcgacctc tagctagagc ttggcgtaat catggtcatt 2640

accaatgctt aatcagtgag gcacctatct cagcgatctg tctatttcgt tcatccatag 2700

ttgcctgact ccccgtcgtg tagataacta cgatacggga gggcttacca tctggcccca 2760

gcgctgcgat gataccgcga gaaccacgct caccggctcc ggatttatca gcaataaacc 2820

agccagccgg aagggccgag cgcagaagtg gtcctgcaac tttatccgcc tccatccagt 2880

ctattaattg ttgccgggaa gctagagtaa gtagttcgcc agttaatagt ttgcgcaacg 2940

ttgttgccat cgctacaggc atcgtggtgt cacgctcgtc gtttggtatg gcttcattca 3000

gctccggttc ccaacgatca aggcgagtta catgatcccc catgttgtgc aaaaaagcgg 3060

ttagctcctt cggtcctccg atcgttgtca gaagtaagtt ggccgcagtg ttatcactca 3120

tggttatggc agcactgcat aattctctta ctgtcatgcc atccgtaaga tgcttttctg 3180

tgactggtga gtactcaacc aagtcattct gagaatagtg tatgcggcga ccgagttgct 3240

cttgcccggc gtcaatacgg gataataccg cgccacatag cagaacttta aaagtgctca 3300

tcattggaaa acgttcttcg gggcgaaaac tctcaaggat cttaccgctg ttgagatcca 3360

gttcgatgta acccactcgt gcacccaact gatcttcagc atcttttact ttcaccagcg 3420

tttctgggtg agcaaaaaca ggaaggcaaa atgccgcaaa aaagggaata agggcgacac 3480

ggaaatgttg aatactcata ttcttccttt ttcaatatta ttgaagcatt tatcagggtt 3540

attgtctcat gagcggatac atatttgaat gtatttagaa aaataaacaa ataggggtca 3600

gtgttacaac caattaacca attctgaaca ttatcgcgag cccatttata cctgaatatg 3660

gctcataaca ccccttgctc atgaccaaaa tcccttaacg tgagttacgc gcgcgtcgtt 3720

ccactgagcg tcagaccccg tagaaaagat caaaggatct tcttgagatc ctttttttct 3780

gcgcgtaatc tgctgcttgc aaacaaaaaa accaccgcta ccagcggtgg tttgtttgcc 3840

ggatcaagag ctaccaactc tttttccgaa ggtaactggc ttcagcagag cgcagatacc 3900

aaatactgtt cttctagtgt agccgtagtt agcccaccac ttcaagaact ctgtagcacc 3960

gcctacatac ctcgctctgc taatcctgtt accagtggct gctgccagtg gcgataagtc 4020

gtgtcttacc gggttggact caagacgata gttaccggat aaggcgcagc ggtcgggctg 4080

aacggggggt tcgtgcacac agcccagctt ggagcgaacg acctacaccg aactgagata 4140

cctacagcgt gagctatgag aaagcgccac gcttcccgaa gggagaaagg cggacaggta 4200

tccggtaagc ggcagggtcg gaacaggaga gcgcacgagg gagcttccag ggggaaacgc 4260

ctggtatctt tatagtcctg tcgggtttcg ccacctctga cttgagcgtc gatttttgtg 4320

atgctcgtca ggggggcgga gcctatggaa aaacgccagc aacgcggcct ttttacggtt 4380

cctggccttt tgctggcctt ttgctcacat gttctttcct gcgttatccc ctgattctgt 4440

ggataaccgt attaccgcct ttgagtgagc tgataccgct cgccgcagcc gaacgaccga 4500

gcgcagcgag tcagtgagcg aggaagcgga aggcgagagt agggaactgc caggcatcaa 4560

actaagcaga aggcccctga cggatggcct ttttgcgttt ctacaaactc tttctgtgtt 4620

gtaaaacgac ggccagtctt aagctcgggc cccctgggcg gttctgataa cgagtaatcg 4680

ttaatccgca aataacgtaa aaacccgctt cggcgggttt ttttatgggg ggagtttagg 4740

gaaagagcat ttgtcagaat atttaagggc gcctgtcact ttgcttgata tatgagaatt 4800

atttaacctt ataaatgaga aaaaagcaac gcactttaaa taagatacgt tgctttttcg 4860

attgatgaac acctataatt aaactattca tctattattt atgatttttt gtatatacaa 4920

tatttctagt ttgttaaaga gaattaagaa aataaatctc gaaaataata aagggaaaat 4980

cagtttttga tatcaaaatt atacatgtca acgataatac aaaatataat acaaactata 5040

agatgttatc agtatttatt atcatttaga ataaattttg tgtcgccctt aattgtgagc 5100

ggataacaat tacgagcttc atgcacagtg gcgttgacat tgattattga ctagttatta 5160

atagtaatca attacggggt cattagttca tagcccatat atggagttcc gcgttacata 5220

acttacggta aatggcccgc ctggctgacc gcccaacgac ccccgcccat tgacgtcaat 5280

aatgacgtat gttcccatag taacgccaat agggactttc cattgacgtc aatgggtgga 5340

gtatttacgg taaactgccc acttggcagt acatcaagtg tatcatatgc caagtacgcc 5400

ccctattgac gtcaatgacg gtaaatggcc cgcctggcat tatgcccagt acatgacctt 5460

atgggacttt cctacttggc agtacatcta cgtattagtc atcgctatta ccatggtgat 5520

gcggttttgg cagtacatca atgggcgtgg atagcggttt gactcacggg gatttccaag 5580

tctccacccc attgacgtca atgggagttt gttttggcac caaaatcaac gggactttcc 5640

aaaatgtcgt aacaactccg ccccattgac gcaaatgggc ggtaggcgtg tacggtggga 5700

ggtctatata agcagagctc tctggctaac tagagaaccc actgcttact ggcttatcga 5760

aattaatacg actcactata gggaagcttc ttgttctttt tgcagaagct cagaataaac 5820

gctcaacttt ggcctcgagg ccaccatgga ctataaggac cacgacggag actacaagga 5880

tcatgatatt gattacaaag acgatgacga taagatggcc ccaaagaaga agcggaaggt 5940

cggtatccac ggagtcccag cagccgtaga tttgagaact ttgggatatt cacagcagca 6000

gcaggaaaag atcaagccca aagtgaggtc gacagtcgcg cagcatcacg aagcgctggt 6060

gggtcatggg tttacacatg cccacatcgt agccttgtcg cagcaccctg cagcccttgg 6120

cacggtcgcc gtcaagtacc aggacatgat tgcggcgttg ccggaagcca cacatgaggc 6180

gatcgtcggt gtggggaaac agtggagcgg agcccgagcg cttgaggccc tgttgacggt 6240

cgcgggagag ctgagagggc ctccccttca gctggacacg ggccagttgc tgaagatcgc 6300

gaagcgggga ggagtcacgg cggtcgaggc ggtgcacgcg tggcacaatg cgctcacggg 6360

agcacccctc aacctgaccc cagaccaggt tgtggccatc gccagcaaca taggtggcaa 6420

gcaggccctc gaaaccgtcc agagactgtt accggttctc tgccaggacc acggcctgac 6480

ccccgaacag gttgtcgcta ttgctagtaa cggcggaggc aaacaggcgc tggaaacagt 6540

tcagcgcctc ttgccggtct tgtgtcaggc ccacggcctg acccccgacc aggttgtcgc 6600

tattgctagt aacggcggag gcaaacaggc gctggaaaca gttcagcgcc tcttgccggt 6660

cttgtgtcag gcccacggcc tgaccccggc ccaggtggtt gcaatcgcgt cacacgatgg 6720

gggaaagcag gccctagaaa ccgttcagcg actcctgccc gtcctgtgcc aggaccacgg 6780

cctgacccca gaccaggttg tggccatcgc cagcaacata ggtggcaagc aggccctcga 6840

aaccgtccag agactgttac cggttctctg ccaggaccac ggcctgaccc cggaacaggt 6900

ggttgcaatc gcgtcacacg atgggggaaa gcaggcccta gaaaccgttc agcgactcct 6960

gcccgtcctg tgccaggccc acggcctgac cccggaccag gtggttgcaa tcgcgtcaca 7020

cgatggggga aagcaggccc tagaaaccgt tcagcgactc ctgcccgtcc tgtgccaggc 7080

ccacggcctg accccagccc aggttgtggc catcgccagc aacataggtg gcaagcaggc 7140

cctcgaaacc gtccagagac tgttaccggt tctctgccag gaccacggcc tgaccccaga 7200

ccaggttgtg gccatcgcca gcaacatagg tggcaagcag gccctcgaaa ccgtccagag 7260

actgttaccg gttctctgcc aggaccacgg cctgaccccg gaacaggtgg ttgcaatcgc 7320

gtcacacgat gggggaaagc aggccctaga aaccgttcag cgactcctgc ccgtcctgtg 7380

ccaggcccac ggcctgaccc cggaccaggt ggttgcaatc gcgtcacacg atgggggaaa 7440

gcaggcccta gaaaccgttc agcgactcct gcccgtcctg tgccaggccc acggcctgac 7500

cccagcccag gttgtggcca tcgccagcaa cataggtggc aagcaggccc tcgaaaccgt 7560

ccagagactg ttaccggttc tctgccagga ccacggcctg accccggacc aggtggttgc 7620

aatcgcgtca cacgatgggg gaaagcaggc cctagaaacc gttcagcgac tcctgcccgt 7680

cctgtgccag gaccacggcc tgaccccaga acaggttgtg gccatcgcca gcaacatagg 7740

tggcaagcag gccctcgaaa ccgtccagag actgttaccg gttctctgcc aggcccacgg 7800

cctgaccccg gaccaggtgg ttgcaatcgc gtcacacgat gggggaaagc aggccctaga 7860

aaccgttcag cgactcctgc ccgtcctgtg ccaggcccac ggcctgaccc cggcccaggt 7920

ggttgcaatc gcgtcacacg atgggggaaa gcaggcccta gaaaccgttc agcgactcct 7980

gcccgtcctg tgccaggacc acggcctgac gcctgagcag gtagtggcta ttgcatccaa 8040

catagggggc agacccgcac tggagtcaat cgtggcccag ctttcgaggc cggaccccgc 8100

gctggccgca ctcactaatg atcatcttgt agcgctggcc tgcctcggcg gacgacccgc 8160

cttggatgcg gtga 8174

<210> 3
<211> 20
<212> DNA
<213> artificial sequence

<220>
<223> primer sequence

<400> 3
acccgcgact ccctattatt 20

<210> 4
<211> 20
<212> DNA
<213> artificial sequence

<220>
<223> primer sequence

<400> 4
tgccttgact gtattgttgg 20

<210> 5
<211> 20
<212> DNA
<213> artificial sequence

<220>
<223> primer sequence

<400> 5
tacctccctg aatggactgc 20

<210> 6
<211> 20
<212> DNA
<213> artificial sequence

<220>
<223> primer sequence

<400> 6

tggttgtgtt ccagtctcca                                                    20

<210>  7
<211>  20
<212>  DNA
<213>  artificial sequence

<220>
<223>  primer sequence

<400>  7
ggtccacttc gtatgctggt                                                    20

<210>  8
<211>  20
<212>  DNA
<213>  artificial sequence

<220>
<223>  primer sequence

<400>  8
ggctgttgct gaggctttac                                                    20